# DEMANDE DE BREVET EUROPEEN

(11) **EP 4 483 961 A2**
(43) Date de publication de la demande: **01.01.2025**
(21) Numéro de dépôt: 24213375.9
(22) Date de dépôt: 22.10.2019
(51) Int. Cl.: A61P 31/00

(54) **EXTRAIT D'ALGUES POUR SON UTILISATION POUR LE TRAITEMENT OU LA PREVENTION DE L'IMMUNOSUPPRESSION POST-TRAUMATIQUE**

(30) Priorité: 22.10.2018 FR 1859721
(62) Demande divisionnaire de: 19801216.3
(71) Demandeur: Amadeite, 56580 Brehan (FR)
(72) Inventeur: ASEHNOUNE, Karim, 44000 NANTES (FR); JACQUELINE, Cédric, 44540 VALLONS-DE-L'ERDRE (FR); BOURAS, Marwan, 44100 NANTES (FR); ROQUILLY, Antoine, 44300 NANTES (FR); NYVALL-COLLÉN, Pi, 29680 ROSCOFF (FR); BALUSSON, Hervé, 56580 BREHAN (FR); DEMAIS, Hervé, 56700 MERLEVENEZ (FR)
(74) Mandataire: Lavoix

(57) **Abrégé**

La présente invention concerne un extrait d'algues de l'ordre des ulvales comprenant des polysaccharides polyanioniques sulfatés et non sulfatés dont le poids moléculaire est inférieur ou égal à 50 kDa, pour son utilisation pour la prévention et/ou le traitement de complications induites par une immunodépression post-traumatique.

## Description

La présente invention concerne un extrait d'algues de l'ordre des ulvales comprenant des polysaccharides polyanioniques sulfatés et non sulfatés dont le poids moléculaire est inférieur ou égal à 50 kDa, pour son utilisation pour la prévention et/ou le traitement de complications induites par une immunodépression post-traumatique.

Le polytraumatisme ou traumatisme sévère s'accompagne d'un syndrome de réponse inflammatoire systémique (SRIS) causé par la libération de composants intracellulaires nommés « *danger-associated molecular patterns »* (DAMP). Ces DAMP vont directement stimuler des récepteurs spécifiques, les *« pattern recognition receptors* », présents sur les cellules de l'immunité, entraînant le relargage de médiateurs de l'inflammation. Pour éviter les complications systémiques d'un SRIS non contrôlé, l'organisme développe précocement une réponse anti-inflammatoire systémique compensatrice (CARS). Le CARS entraîne une immunodépression post-traumatique, de durée et d'amplitude variable. Cette immunodépression est « physiologique » au départ et présente chez tous les patients. L'immunodépression devient pathologique si elle perdure, entraînant alors des infections post-traumatiques (pneumopathies essentiellement) qui sont la première cause de complications en réanimation (Roquilly et al., 2015, Réanimation, 24 :S285-S290).

Le traumatisme crânien est une cause majeure de morbi-mortalité, avec un taux de mortalité global des victimes variant selon les estimations de 5 à 25 pour 100 000 patients. Environ un tiers des facteurs de morbi-mortalité sont des complications évitables, survenant en réanimation (infections, événements thrombo-emboliques), principalement due aux complications respiratoires. La morbidité et les coûts de santé occasionnés par les pneumonies acquises sous ventilation mécanique (PAVM) en font l'une des complications liées aux soins les plus. Entre 40 et 60% des patients atteints de traumatisme crânien grave hospitalisés en réanimation présenteront une PAVM, dont la moitié est causée par *Staphyloccocus aureus* sensible à la méticiline (SAMS). Au cours de son hospitalisation en réanimation, un patient traumatisé crânien souffrant de PAVM subira plus d'agressions cérébrales d'origine systémique (fièvre, hypotension, hypoxie, hypercapnie) ; la durée de ventilation mécanique sera également prolongée, ainsi que le séjour en réanimation. Tous ces facteurs contribuent d'une part à l'apparition de nouvelles lésions cérébrales ischémiques sur hypoxie et d'autre part à un retard de prise en charge post-réanimatoire (rééducation...). Le développement d'une PAVM chez un patient traumatisé crânien est donc un facteur de risque indépendant de moins bon devenir neurologique à long terme. La susceptibilité des patients traumatisés crânien aux infections nosocomiales et notamment aux PAVM est due à l'apparition d'une immunodépression post-traumatique.

Au niveau cellulaire, cette immunodépression post-traumatique se déroule à plusieurs niveaux :
1. Une altération de la capacité de présentation de l'antigène par les cellules présentatrices d'antigène (CPA), principalement Cellules Dendritiques (CDs) et monocytes. Les CDs ont un rôle central de capture et de présentation de l'antigène, ainsi que d'activation des lymphocytes (T et NK) via la sécrétion de cytokines pro-inflammatoires (Interleukine-12).
2. Une diminution de la capacité de sécrétion de cytokines pro-inflammatoires. La coopération entre les CPA et les lymphocytes en cas d'agression pulmonaires aigüe est essentielle. Ainsi, une altération de la production d'IL-12, conséquence du traumatisme crânien induira une diminution de sécrétion de cytokines pro-inflammatoires telles que l'INF γ par les cellules lymphoïdes (Spolarics et al., 2003, Crit. Care Med.;31(6):1722-9). D'autre part, l'augmentation de la sécrétion de cytokines anti-inflammatoires (IL-10) via l'hyper-activation sympathique contribue à approfondir cet état d'immunodépression (Roquilly et al., 2014, Crit. Care Med., 42(12):e752-61). L'altération de cette « boucle » de la réponse immunitaire innée provoque donc une diminution des capacités de l'organisme à lutter contre les infections secondaires.
3. Un épuisement des lymphocytes T (« T cell exhaustion »), phénomène également retrouvé dans l'immunodépression post-septique. Cet épuisement correspond à la perte progressive de fonctions pro-inflammatoires des lymphocytes en présence d'une charge antigénique élevée. Ce phénomène permettrait d'expliquer en partie la forte incidence des PAVM chez les patients traumatisés crâniens. Ce phénomène d'« exhaustion » et la lymphopénie qui en résulte est un facteur de risque de mortalité chez les patients polytraumatisés (Heffernan et al., 2012, Crit. Care., 20;16(1):R12). Cette lymphopénie persiste d'ailleurs au-delà de 6 mois chez la plupart des patients.

Dans le but de corriger cette immunodépression post-traumatique, de nombreuses thérapeutiques ont été testées au cours des dernières années. Elles visaient soit à limiter le SIRS initial (et donc le CARS) notamment par l'utilisation de glucocorticoïdes à faible dose, soit à restaurer les capacités de présentation de l'antigène ou de sécrétions de cytokines par l'utilisation de glucans, d'IFN γ, de GM-CSF ou d'Interleukine 12.

Les « toll-like-receptors » (TLR) sont impliqués dans la reconnaissance des molécules de danger (DAMPS). Ces récepteurs sont situés à la surface des cellules de l'immunité innée. Dix TLR différents ont été identifiés chez l'Homme, chacun d'entre eux reconnaissant un ou plusieurs DAMPS. Après reconnaissance avec son ligand, le récepteur TLR peut activer deux grandes voies de signalisation intracellulaire aboutissant à l'activation i) du facteur de transcription nucléaire-κB (NF-κB, voie MyD88 dépendante) conduisant à la fixation sur le promoteur des gènes des cytokines pro-inflammatoires (IL-12, TNFα), et ii) des facteurs de transcription nucléaires de la famille des IRF (Interferon regulatory factor : IRF, voie TRIF dépendante). Les récepteurs les plus étudiés sont le TLR4, impliqué dans la reconnaissance du lipopolysaccharide (LPS) composant la paroi des bacilles gram négatif et le TLR2 reconnaissant quant à lui l'acide lipotéichoïque des bactéries gram positif.

Les agonistes TLR ont été suggérés comme une voie de recherche possible dans le but de restaurer les fonctions immunitaires « paralysées » par le traumatisme et de lutter contre les infections secondaires (Hedayat et al., 2011, Lancet Infect Dis.;11(9):702-12).

Il a été montré que l'administration de lipide A monophosphoryle (MPLA), un dérivé non toxique du LPS connu pour avoir une activité agoniste de TLR4, prévenait la mortalité dans un modèle de pneumonie chez la souris immunodéprimée post choc hémorragique (Roquilly et al. 2010, PLoS One 7;5(10):e13228 ; demande de brevet internationale WO2011080126). Le mécanisme d'action mis en jeu n'a toutefois pas été élucidé. Dans ce modèle de pneumonie chez la souris immunodéprimée post choc hémorragique, il a aussi été montré que MPLA restaure partiellement la fonction des CDs, prévient la surexpression d'ARNm de l'IL-10 par les cellules NK, et que la stimulation directe des CDs par MPLA diminue la mortalité, sans qu'une stimulation directe des cellules NK ne soit requise à cette réponse immune contre la pneumonie (Roquilly et al., 2013, Eur Respir J.;42(5):1365-78). Comme admis par les auteurs eux-mêmes, il n'a toutefois pas été montré que cet effet de MPLA soit médié par TLR4, en particulier par des récepteurs TLR4 exprimés à la surface des CDs ou cellules NK.

Les composés à base d'algues marines sont utilisés dans différents domaines allant de l'industrie pharmaceutique à la microbiologie. Leurs composants biologiquement actifs comprennent principalement des peptides, des acides gras polyinsaturés et des polysaccharides. La paroi cellulaire des algues marines est riche en polysaccharides sulfatés tels que les ulvans dans les algues vertes. Récemment, il a été montré qu'une fraction purifiée d'ulvans extraits à partir d'une algue verte de type *Ulva* avait une activité immunomodulatrice, notamment *in vitro* sur des cellules intestinales porcines IPEC-1 (demande de brevet WO2015071497). Dans cette lignée cellulaire IPEC-1, l'extrait d'algues stimule la sécrétion de cytokines pro-inflammatoires (IL1β, IL-6, IL-8, TNFα ...) *in vitro* (Berri et al., 2016, Journal of Applied Phycology, 28(5): 2999-3008), via la voie TLR4/NF-κB (Berri et al., 2017, Algal Research, 28, 39-47).

Les inventeurs ont cherché à déterminer si un extrait d'algues de l'ordre des ulvales, en particulier d'algues vertes de type *Ulva,* comprenant des polysaccharides polyanioniques sulfatés et non sulfatés dont le poids moléculaire est inférieur ou égal à 50 kDa, pourrait prévenir ou traiter l'immunodépression post-traumatique, en particulier ses complications respiratoires. Les effets de cet extrait sur l'immunité pulmonaire muqueuse ont en particulier été évalués dans un modèle murin de traumatisme crânien (Flierl et al., 2009, Nat Protoc.;4(9):1328-37), suivi d'une pneumonie à SAMS (*Staphyloccocus aureus* sensible à la méticilline).

Il a ainsi été montré, dans le modèle murin d'infection pulmonaire bactérienne post-traumatique, que l'extrait d'algues :
- limite la dissémination bactérienne systémique (splénique), sans que cet effet ne soit liée à une action antibactérienne directe de l'extrait, indiquant ainsi une restauration des fonctions immunitaires paralysées par le traumatisme ;
- n'augmente pas la proportion de cellules de l'immunité innée productrices de cytokines pro-inflammatoires dans le poumon (CDs productrices d'IL-12, macrophages producteurs de TNFα, NK ou LT producteurs d'INFy) ;
- augmente le nombre de cellules NK totales et le nombre de cellules NK produisant de l'INFy, sans augmentation du pourcentage de cellules NK INFγ⁺, dans le poumon (lieu de l'infection), mais pas dans la rate. Cet effet contrebalance donc la diminution du nombre de cellules NK totales et de cellules NK INFγ⁺, dans le poumon, induite par le traumatisme crânien ;
- n'a plus d'effet sur la dissémination bactérienne systémique lorsque la souris a été déplétée en cellules NK (>95% de déplétion en NK pulmonaires) ;
- n'induit, dans les poumons, aucune augmentation du taux des chimiokines impliquées dans le chimiotactisme des cellules NK

Lorsque l'infection pulmonaire bactérienne chez la souris est réalisée en l'absence de traumatisme crânien (et donc d'immunodépression), l'extrait d'algues n'induit pas de stimulation des cellules de l'immunité innée, que ce soit via une stimulation de la sécrétion d'IL-12 par les CDs, de TNFα par les macrophages ou d'INFγ par les cellules NK ou LT, ou via une stimulation de l'expression membranaire du CMH II par les CDs, et ne limite pas la dissémination bactérienne.

Par ailleurs, la stimulation directe *in vitro de* cellules NK pulmonaires de souris naïves ou traumatisées, par l'extrait d'algue, n'induit aucune augmentation de la production d'interféron γ ou du marqueur KLRG1 d'activation des cellules NK.

Ces résultats montrent donc l'utilité d'un extrait d'algues de l'ordre des ulvales, en particulier d'algues vertes de type *Ulva,* comprenant des polysaccharides polyanioniques sulfatés et non sulfatés dont le poids moléculaire est inférieur ou égal à 50 kDa, pour prévenir ou traiter l'immunodépression post-traumatique.

### Résumé de l'invention

L'invention concerne un extrait d'algues de l'ordre des ulvales comprenant des polysaccharides polyanioniques sulfatés et non sulfatés dont le poids moléculaire est inférieur ou égal à 50 kDa, pour son utilisation pour la prévention et/ou le traitement d'une complication induite par une immunodépression post-traumatique.

Selon un mode de réalisation, la complication induite est une complication septique de l'immunodépression post-traumatique. Selon certains modes de réalisation, la complication sceptique est une infection nosocomiale, notamment une infection nosocomiale sélectionnées dans le groupe constitué des pneumopathies, telles que les pneumonies acquises sous ventilation mécanique (PAVMs), des infections urinaires, des infections sur cathéters veineux centraux, des infections bactériennes cérébroméningées telles méningites empyèmes et abcès cérébraux.

Selon un mode de réalisation, l'immunodépression post-traumatique est consécutive à un ou plusieurs traumatismes sévères, en particulier un traumatisme crânien sévère.

L'extrait d'algues de l'ordre des ulvales est de préférence un extrait d'algues vertes de type *Ulva.*

Selon un mode de réalisation, lesdits polysaccharides polyanioniques sulfatés et non sulfatés de poids moléculaire inférieur ou égal à 50 kDa ont un poids moléculaire inférieur à 15 kDa, et de préférence supérieur à 500 Da. De préférence, l'extrait d'algues ne comprend pas de polysaccharides polyanioniques sulfatés ou non sulfatés dont le poids moléculaire est supérieur à 15 kDa.

Selon un mode de réalisation, l'extrait d'algue comprend :
- du mannose ; et/ou
- de l'arabinose ; et/ou
- du galactose; et/ou
- du glucose; et/ou
- du rhamnose; et/ou
- du xylose; et/ou
- de l'acide glucuronique.

Selon un mode de réalisation, l'extrait d'algue comprend :
- de 10 à 50 % de carbone ;
- de 1 à 10% d'hydrogène ;
- de 1 à 5 % d'azote ;
- de 20 à 50 % d'oxygène ; et
- de 1 à 15 % de soufre ;
en pourcentage massique de la matière sèche totale de l'extrait d'algues.

Selon un autre mode de réalisation, l'extrait d'algues est obtenu par un procédé de préparation dans lequel:
a) les algues sont lavées et dessablées ;
b) lesdites algues sont broyées ;
c) la phase solide du broyat est séparée de sa phase liquide ;
d) ladite phase liquide est clarifiée ;
e) le jus obtenu à l'étape d) est ultrafiltré sur une membrane de 50 kDa ou moins; et
f) le jus de filtration obtenu à l'étape e) est concentré puis séché.

### Description détaillée

Selon un aspect, l'invention concerne un extrait d'algues de l'ordre des ulvales comprenant des polysaccharides polyanioniques sulfatés et non sulfatés dont le poids moléculaire est inférieur ou égal à 50 kDa, pour son utilisation pour la prévention et/ou le traitement de complications induites par une immunodépression post-traumatique.

Selon un aspect, l'invention concerne une méthode de traitement prophylactique ou thérapeutique de complications induites par une immunodépression post-traumatique chez un sujet le nécessitant, la méthode comprenant l'administration audit sujet d'une quantité efficace d'un extrait d'algues de l'ordre des ulvales comprenant des polysaccharides polyanioniques sulfatés et non sulfatés dont le poids moléculaire est inférieur ou égal à 50 kDa.

### Extrait d'algues de l'ordre des ulvales

Un extrait d'algues comprenant des polysaccharides polyanioniques sulfatés et non sulfatés dont le poids moléculaire est inférieur ou égal à 50 kDa a notamment été décrit dans la demande de brevet WO2015071497.

L'extrait d'algues de l'ordre des ulvales comprenant des polysaccharides polyanioniques sulfatés et non sulfatés dont le poids moléculaire est inférieur ou égal à 50 kDa est en particulier un extrait d'algues vertes de type *Ulva.*

Par « algues vertes de type *Ulva* » on entend les algues vertes regroupées dans le genre *Ulva,* de la famille des Ulvaceae, de l'ordre des ulvales. On peut notamment citer les espèces et sous-espèces suivantes : *Ulva acanthophora, Ulva anandii, Ulva angusta, Ulva arasakii, Ulva armoricana, Ulva atroviridis, Ulva attenuata, Ulva beytensis, Ulva bifrons, Ulva brevistipitata, Ulva bulbosa, Ulva burmanica, Ulva byssoides, Ulva californica, Ulva chaetomorphoides, Ulva clathrata, Ulva coccinea, Ulva compressa, Ulva conglobata, Ulva cornucopiae, Ulva cornuta, Ulva covelongensis, Ulva crassa, Ulva crassimembrana, Ulva curvata, Ulva dactylifera, Ulva denticulata, Ulva elegans, Ulva elminthoides, Ulva enteromorpha, Ulva erecta, Ulva expansa, Ulva fasciata, Ulva fenestrata, Ulva flexuosa, Ulva gelatinosa, Ulva geminoidea, Ulva gigantea, Ulva grandis, Ulva hendayensis, Ulva hookeriana, Ulva hopkirkii, Ulva indica, Ulva intestinalis, Ulva intestinaloides, Ulva intricata, Ulva intybacea, Ulva javanica, Ulva kylinii, Ulva lactuca, Ulva lactucaefolia, Ulva laetevirens, Ulva laingii, Ulva linearis, Ulva lingulata, Ulva linkiana, Ulva linza, Ulva lippii, Ulva litoralis, Ulva littorea, Ulva lobata, Ulva lubrica, Ulva marginata, Ulva micrococca, Ulva myriotrema, Ulva neapolitana, Ulva nematoidea, Ulva ohnoi, Ulva olivacea, Ulva olivaceum, Ulva pacifica, Ulva papenfussii, Ulva paradoxa, Ulva parva, Ulva parvula, Ulva patengensis, Ulva percursa, Ulva perfusa, Ulva phyllosa, Ulva popenguinensis, Ulva porrifolia, Ulva procera, Ulva profunda, Ulva proliféra, Ulva pseudocurvata, Ulva pseudolinza, Ulva pulchra, Ulva purpurascens, Ulva quilonensis, Ulva radiata, Ulva ralfsii, Ulva ranunculata, Ulva reticulata, Ulva rhacodes, Ulva rigida, Ulva rotundata, Ulva rubens, Ulva saifullahii, Ulva scagelii, Ulva scandinavica, Ulva sericea, Ulva serrata, Ulva simplex, Ulva sorensenii, Ulva spinulosa, Ulva stenophylla, Ulva stipitata, Ulva sublittoralis, Ulva subulata, Ulva taeniata, Ulva tenera, Ulva tetragona, Ulva torta, Ulva tuberosa, Ulva umbilicata, Ulva uncialis, Ulva uncinata, Ulva usneoides, Ulva utricularis, Ulva utriculosa, Ulva uvoides, Ulva ventricosa.*

L'extrait d'algues ci-dessus comprend des polysaccharides polyanioniques sulfatés et non sulfatés dont le poids moléculaire est inférieur ou égal à 50 kDa. Plus particulièrement, l'extrait d'algues comprend des polysaccharides polyanioniques sulfatés et non sulfatés de poids moléculaire inférieur ou égal à 50 kDa, dont le poids moléculaire est inférieur à 40, 30, 20 ou 15 kDa. Plus particulièrement encore, les polysaccharides polyanioniques sulfatés et non sulfatés de l'extrait d'algues ont un poids moléculaire inférieur ou égal à 15 kDa. De préférence, les polysaccharides polyanioniques sulfatés et non sulfatés de l'extrait d'algues ont un poids moléculaire supérieur à 500 Da, de préférence supérieur à 750 Da.

Selon un mode réalisation de l'invention, l'extrait d'algues ne comprend pas de polysaccharides polyanioniques sulfatés ou non sulfatés dont le poids moléculaire est supérieur à 50 kDa (ou supérieur à 40, 30, 20 ou 15 kDa lorsque les polysaccharides sulfatés et non sulfatés sont définis par un poids moléculaire inférieur à 40, 30, 20 ou 15 kDa, respectivement). Ainsi, selon un mode de réalisation, lorsque l'extrait d'algues comprend des polysaccharides polyanioniques sulfatés et non sulfatés dont le poids moléculaire est inférieur ou égal à 15 kDa, l'extrait d'algues ne comprend pas de polysaccharides polyanioniques sulfatés ou non sulfatés dont le poids moléculaire est supérieur à 15 kDa.

Un dalton (Da) est une unité massique définie comme étant égal à un douzième de la masse d'un atome de carbone 12, masse qui s'avérera ensuite estimée à partir d'un mélange de plusieurs isotopes (principalement carbone 12 et carbone 13, possédant respectivement 6 et 7 neutrons en plus des 6 protons de tout atome de carbone). Un dalton est, avec une assez bonne précision, la masse d'un atome d'hydrogène, la valeur exacte étant 1,00794 uma (unité de masse atomique). Le kilodalton (kDa) est égal à 1000 Da. Dans le cadre de la présente invention, les masses mentionnées en kDa sont déterminées par toute méthode usuellement employée par l'homme du métier, en particulier les masses des polysaccharides polyanioniques sulfatés et non sulfatés des extraits d'algues pourront être discriminées par ultrafiltration sur des membranes laissant uniquement filtrer des molécules de tailles prédéterminées.

Selon un mode de réalisation les polysaccharides polyanioniques sulfatés et non sulfatés dont le poids moléculaire est supérieur à 50 kDa, tel que décrit ci-dessus, ont un degré de polymérisation supérieur à 5 unités oses.

Dans l'extrait d'algues tel que défini ci-dessus, les polysaccharides comprennent du mannose et/ou de l'arabinose, préférentiellement du mannose. Plus particulièrement encore, l'extrait d'algues comprend au moins 0.005% de mannose et/ou au moins 0.005% d'arabinose, en poids par rapport au poids de la matière sèche totale de l'extrait d'algues, notamment au moins au 0,01 % de mannose et/ou au moins 0,01 % d'arabinose. Préférentiellement l'extrait d'algues comprend au moins 0,005% de mannose.

Encore plus particulièrement, l'extrait d'algues comprend du mannose en une quantité allant de 0,005 à 0,5%, par exemple de 0,005 à 0,2% ou de 0,15 à 0,5% et/ou de l'arabinose en une quantité allant de 0,005 à 0,5%, en poids par rapport au poids de la matière sèche totale de l'extrait d'algues, préférentiellement du mannose en une quantité allant de 0,005 à 0,5%, par exemple de 0,005 à 0,2% ou de 0,15 à 0,5% en poids par rapport au poids de la matière sèche totale de l'extrait d'algues.

Encore plus particulièrement, l'extrait d'algues comprend du mannose en une quantité allant de 0,01 à 0,5%, par exemple de 0,01 à 0,2% ou de 0,2 à 0,5% et/ou de l'arabinose en une quantité allant de 0,01 à 0,5%, en poids par rapport au poids de la matière sèche totale de l'extrait d'algues, notamment du mannose en une quantité allant de 0,03 à 0,45%, par exemple de 0,03 à 0,15% ou de 0,15 à 0,45% et/ou de l'arabinose en une quantité allant de 0,01 à 0,2%.

Préférentiellement, l'extrait d'algues comprend du mannose en une quantité allant de 0,01 à 0,50%, par exemple de 0,01 à 0,20% ou de 0,20 à 0,5%, notamment du mannose en une quantité allant de 0,03 à 0,45%, par exemple de 0,03 à 0,15% ou de 0,15 à 0,45%%, en poids par rapport au poids de la matière sèche totale de l'extrait d'algues.

Selon un mode de réalisation, l'extrait d'algues comprend :
- du galactose; et/ou
- du glucose; et/ou
- du rhamnose; et/ou
- du xylose; et/ou
- de l'acide glucuronique.

Plus particulièrement, l'extrait d'algues comprend:
- de 0,05 à 0,5% de galactose, notamment de 0,1 à 0,4%; et/ou
- de 0,005 à 0,5% de glucose, en particulier de 0,005 à 0,05% notamment de 0,01 à 0,03%, ou de 0,05 à 0,5% ; et/ou;
- de 2 à 15 % de rhamnose, notamment de 5 à 10%; et/ou
- de 0,1 à 1% de xylose, notamment de 0,3 à 0,7%; et/ou
- de 1 à 7% d'acide glucuronique notamment de 1 à 5%;
en poids par rapport au poids de la matière sèche totale de l'extrait d'algues.

Ainsi, selon un mode de réalisation l'extrait d'algues comprend:
- du mannose ; et/ou
- de l'arabinose ; et/ou
- du galactose; et/ou
- du glucose; et/ou
- du rhamnose; et/ou
- du xylose; et/ou
- de l'acide glucuronique.

On peut plus particulièrement citer par exemple l'extrait d'algues comprenant :
- de 0,01 à 0,5% de mannose, par exemple de 0,01 à 0,2%, notamment de 0,03 à 0,15% ou de 0,2 à 0,5% ; et/ou
- de 0,01 à 0,5% d'arabinose, notamment de 0,01 à 0,2% ; et/ou
- de 0,05 à 0,5% de galactose, notamment de 0,1 à 0,4%; et/ou
- de 0,005 à 0,5% de glucose, en particulier de 0,005 à 0,05%, notamment de 0,01 à 0,03%, ou de 0,05 à 0,5%; et/ou
- de 2 à 15 % de rhamnose, notamment de 5 à 10%; et/ou
- de 0,1 à 1% de xylose, notamment de 0,3 à 0,7%; et/ou
- de 1 à 7% d'acide glucuronique, notamment de 1 à 5%;
en poids par rapport au poids de la matière sèche totale de l'extrait d'algues.

On peut encore plus particulièrement citer par exemple l'extrait d'algues comprenant :
- 0,09% de mannose; et/ou
- 0,1% d'arabinose ; et/ou
- 0,3% de galactose; et/ou
- 0,02% de glucose ; et/ou
- 8,1% de rhamnose; et/ou
- 0,5% de xylose; et/ou
- 2,6% d'acide glucuronique;
en poids par rapport au poids de la matière sèche totale de l'extrait d'algues.

On peut également citer par exemple l'extrait d'algues comprenant :
- 0,3% de mannose; et/ou
- 0,2% de galactose; et/ou
- 0,4% de glucose ; et/ou
- 7,9% de rhamnose; et/ou
- 0,5% de xylose; et/ou
- 4,9% d'acide glucuronique;
en poids par rapport au poids de la matière sèche totale de l'extrait d'algues.

Selon un mode de réalisation, l'extrait d'algues comprenant des polysaccharides polyanioniques sulfatés et non sulfatés dont le poids moléculaire est inférieur ou égal à 50 kDa, tel que décrit ci-dessus, comprend :
- de 10 à 50 % de carbone ;
- de 1 à 10% d'hydrogène ;
- de 1 à 5 % d'azote ;
- de 20 à 50 % d'oxygène ; et
- de 1 à 15 % de soufre ;
en pourcentage massique de la matière sèche totale de l'extrait d'algues.

Plus particulièrement encore, l'extrait d'algues comprend :
- de 15 à 30 % de carbone ;
- de 3 à 6% d'hydrogène ;
- de 1 à 3 % d'azote ;
- de 25 à 40 % d'oxygène ; et
- de 2,5 à 10 % de soufre ;
en pourcentage massique de la matière sèche totale de l'extrait d'algues.

Selon un autre mode de réalisation de l'invention, l'extrait d'algues comprend :
- de 10 à 50 % de carbone ;
- de 1 à 10% d'hydrogène ;
- de 0,5 à 5 % d'azote ;
- de 20 à 60 % d'oxygène ; et
- de 1 à 15 % de soufre ;
en pourcentage massique de la matière sèche totale de l'extrait d'algues.

Les autres éléments chimiques présents dans la matière sèche de l'extrait sont notamment représentés par les minéraux (Ca, K, Na, Mg, AI, CI, I, P, Fe, etc).

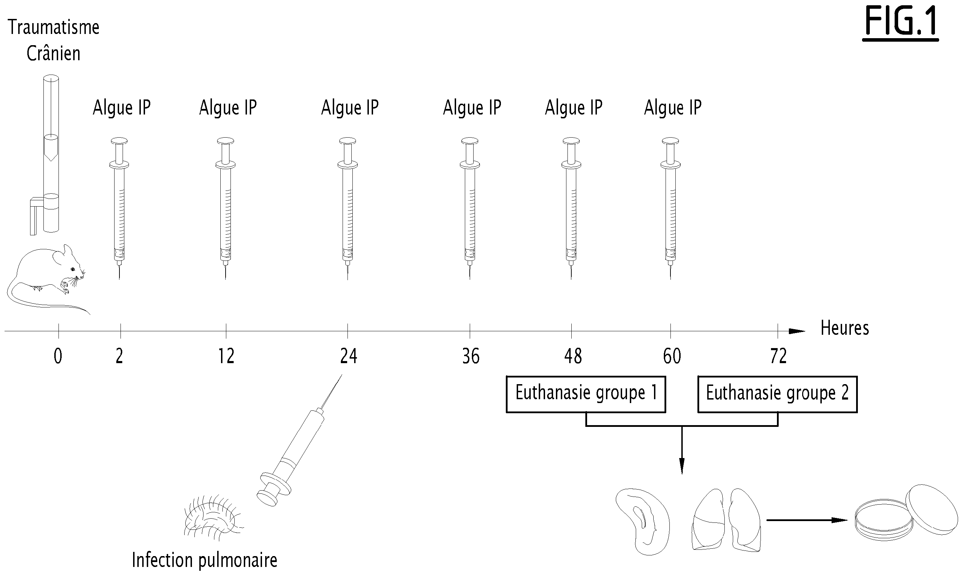

Plus particulièrement, l'extrait d'algues tel que décrit dans le cadre de la présente invention est caractérisé par le spectre RMN ¹H présenté en Figure 1.

Ce spectre RMN ¹H a été enregistré à 298 K sur un spectromètre Bruker Avance 500 équipé d'une sonde cryogénique inverse 5 mm ¹H/¹³C/¹⁵N TCl. Avant l'analyse, les échantillons ont été dissous dans 99,97% d'atome de D₂O. Les déplacements chimiques sont exprimés en ppm par rapport à un étalon externe (acide triméthylsilylpropionique). Aucune suppression du signal HOD n'a été réalisée.

L'extrait d'algues de l'ordre des ulvales comprenant des polysaccharides polyanioniques sulfatés et non sulfatés dont le poids moléculaire est inférieur ou égal à 50 kDa, tel que décrit ci-dessus, est susceptible d'être obtenu, ou est obtenu, par un procédé de préparation dans lequel:
a) les algues sont lavées et dessablées ;
b) lesdites algues sont broyées ;
c) la phase solide du broyat est séparée de sa phase liquide ;
d) ladite phase liquide est clarifiée ;
e) le jus obtenu à l'étape d) est ultrafiltré sur une membrane de 50 kDa ou moins; et
f) le jus de filtration obtenu à l'étape e) est concentré puis séché.

Selon un mode de réalisation, le procédé de préparation comprend en outre une étape de congélation suivie d'une décongélation, entre l'étape a) de lavage/dessablage et l'étape b) de broyage.

En particulier, pour la mise en oeuvre du procédé tel qu'indiqué dans le cadre de la présente invention, dans l'étape a) de celui-ci, les algues sont lavées à l'eau douce.

Elles peuvent être dessablées par tout moyen mis à disposition de l'homme du métier.

Lesdites algues sont ensuite broyées, notamment au moyen d'un broyeur, comme par exemple un affineur ou un cutteur.

Par la suite, la phase solide du broyat, le marc, est séparée de sa phase liquide, le jus, par pressage du broyat, par exemple à l'aide d'une presse à bande ou à plateaux, ou par centrifugation.

Par « jus », on entend le jus cytoplasmique qui inclue la structure pariétale de la double structure des cellules des algues.

La phase liquide obtenue est ensuite clarifiée, par exemple avec un clarificateur à assiettes, ou par centrifugation, décantation ou filtration (par exemple à poche ou à plaque).

Le jus obtenu est ensuite ultrafiltré.

Selon un mode de réalisation pour la mise en oeuvre du procédé tel qu'indiqué dans le cadre de la présente invention, l'ultrafiltration est réalisée sur une membrane de 50 kDa ou moins, notamment sur une membrane de 40, 30, 20 ou 15 kDa. Plus particulièrement, la membrane est une membrane de 15 kDa ou moins.

Cette membrane peut être par exemple une membrane de céramique ou une membrane organique. Plus particulièrement, la membrane est une membrane de céramique.

Le jus de filtration obtenu peut ensuite être concentré, par exemple par osmose inverse, évaporation ou précipitation, puis séché par exemple par lyophilisation ou atomisation.

Optionnellement, l'extrait obtenu pourra ensuite être broyé de nouveau, afin d'obtenir une poudre homogène en terme de granulométrie.

Selon un de ces aspects, le procédé se déroule en partie à température ambiante. Par température ambiante, on entend une température comprise entre 5 et 25°C.

Selon un autre de ces aspects, le procédé se déroule en partie à une température comprise entre 4 et 10°C, ceci afin d'éviter les développements microbiens.

Selon un mode de réalisation pour la mise en oeuvre du procédé tel qu'indiqué dans le cadre de la présente invention, l'extrait d'algues obtenu à l'étape f) du procédé susmentionné est purifié, par exemple par ultrafiltration, en particulier sur une cassette d'ultrafiltration, notamment afin d'éliminer la partie minérale.

Le procédé de préparation de l'extrait d'algues tel que décrit ci-dessus n'utilise pas de solvants, en particulier organiques.

De préférence, l'extrait d'algues de l'ordre des ulvales comprenant des polysaccharides polyanioniques sulfatés et non sulfatés dont le poids moléculaire est inférieur ou égal à 50 kDa présente un ou plusieurs des effets suivants:
- il limite la dissémination bactérienne systémique, de préférence par un mécanisme médié par les cellules NK ;
- il augmente le nombre de cellules NK totales et le nombre de cellules NK produisant de l'INFy, de préférence sans augmentation du pourcentage de cellules NK INFγ⁺, au lieu de l'infection ;
- il n'augmente pas la proportion de cellules de l'immunité innée productrices de cytokines pro-inflammatoires au lieu de l'infection (notamment CDs productrices d'IL-12 et/ou macrophages producteurs de TNFα et/ou NK ou LT producteurs d'INFy) ;
- il n'a pas d'effet antibactérien direct, notamment sur les *Staphyloccocus aureus* sensibles à la méticilline ;
- il n'induit pas d'augmentation de la production d'interféron γ par les cellules NK, par stimulation directe *in vitro* ;
- il n'induit pas d'augmentation de l'expression du marqueur KLRG1 les cellules NK, par stimulation directe *in vitro* ;
- il n'induit pas d'augmentation du taux de la chimiokine CXCL-1 et/ou CXCL-2 au lieu de l'infection.

### Composition pharmaceutique

L'extrait d'algues de l'ordre des ulvales comprenant des polysaccharides polyanioniques sulfatés et non sulfatés dont le poids moléculaire est inférieur ou égal à 50 kDa est de préférence formulé dans une composition pharmaceutique ou un médicament.

Une composition pharmaceutique ou médicament pour la mise en oeuvre de l'invention comprend typiquement l'extrait d'algues de l'ordre des ulvales comprenant des polysaccharides polyanioniques sulfatés et non sulfatés dont le poids moléculaire est inférieur ou égal à 50 kDa, et un excipient pharmaceutiquement acceptable.

Les excipients pharmaceutiquement acceptables utilisés pour la préparation d'un médicament ou d'une composition pharmaceutique comprenant un extrait d'algues pour son utilisation selon l'invention sont choisis selon la forme pharmaceutique et le mode d'administration souhaité, parmi les excipients habituels qui sont connus de l'homme du métier.

Pour une administration orale, sublinguale, sous-cutanée, intramusculaire, intraveineuse, topique, locale, intrapéritonéale, intratrachéale, intranasale, transdermique ou rectale, l'extrait d'algues tel que défini ci-dessus, peut être administré sous forme de doses unitaires, en mélange avec des excipients pharmaceutiques classiques.

Les formes d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, parentérale telle que transdermique, intrapéritonéale, intratrachéale, sous-cutanée, intramusculaire ou intraveineuse, les formes d'administration rectale et les implants.

Lorsqu'une composition solide sous forme de comprimés est préparée, l'ingrédient actif principal peut être mélangé avec un véhicule pharmaceutique, tel que la gélatine, l'amidon, le lactose, le stéarate de magnésium, le talc, la gomme arabique ou analogues.

Les comprimés peuvent également être enrobés avec du saccharose, un dérivé cellulosique, ou d'autres matières appropriées ou encore ils peuvent être traités de telle sorte qu'ils aient une activité prolongée ou retardée et qu'ils libèrent d'une façon continue une quantité prédéterminée de principe actif.

Une préparation sous forme de gélules peut par exemple être obtenue en mélangeant l'ingrédient actif avec un diluant et en versant le mélange obtenu dans des gélules molles ou dures.

Dans un mode de réalisation, l'extrait d'algues, le médicament ou la composition pharmaceutique pour son utilisation selon la présente invention est destiné à une administration orale. Dans un autre mode de réalisation, l'extrait d'algues, le médicament ou la composition pharmaceutique pour son utilisation selon la présente invention est destiné à une administration parentérale.

Les médicaments ou compositions pharmaceutiques comprenant un extrait d'algues pour son utilisation selon l'invention, peuvent aussi se présenter sous forme liquide, par exemple, sous forme de solutions, d'émulsions, de suspensions ou de sirops, et notamment sous une forme adaptée pour une administration orale ou intranasale, par exemple. Les supports liquides appropriés peuvent être, par exemple, de l'eau, des solvants organiques tels que le glycérol ou les glycols, de même que leurs mélanges, dans des proportions variées, dans l'eau.

Une préparation sous forme de sirop ou d'élixir ou pour l'administration sous forme de gouttes peut également contenir l'ingrédient actif conjointement avec un édulcorant, acalorique par exemple, du méthylparabène et du propylparabène comme antiseptique, ainsi qu'un agent donnant du goût et un colorant approprié.

Les poudres ou les granules dispersibles dans l'eau peuvent par exemple contenir l'ingrédient actif en mélange avec des agents de dispersion ou des agents mouillants, ou des agents de mise en suspension, comme la polyvinylpyrrolidone, de même qu'avec des édulcorants ou des correcteurs de goût.

En général, dans le cadre de la présente invention, la dose journalière de l'extrait d'algues sera la dose efficace la plus faible de l'extrait d'algues capable de produire un effet prophylactique et/ou thérapeutique sur une immunodépression post-traumatique.

Par « dose efficace », on désigne toute quantité d'une composition qui permet d'observer l'effet recherché, ici un effet immunostimulateur.

Selon un de ses aspects, un extrait d'algues pour son utilisation selon l'invention est utilisé dans une composition telle que susmentionnée pour une administration à une dose chez l'humain comprise entre 0,1 et 100 mg/kg, encore plus particulièrement entre 0,5 et 60 mg/kg, par exemple entre 1 et 20 mg/kg ou entre 5 et 30 mg/kg, ou à une dose chez l'animal comprise entre 1 et 200 mg/kg, plus particulièrement entre 1 et 100 mg/kg, encore plus particulièrement entre 2 et 45 mg/kg ou entre 10 et 60 mg/kg.

### Traitement prophylactique ou thérapeutique de complications induites par une immunodépression post-traumatique

Dans le contexte de l'invention, le terme "traiter" ou "traitement" signifie supprimer, alléger ou empêcher la progression d'un désordre ou d'un ou plusieurs symptômes liés à ce désordre. Un traitement "prophylactique" ou "préventif" signifier prévenir l'apparition d'un tel désordre ou d'un ou plusieurs symptômes liés à ce désordre.

Un sujet pour le traitement prophylactique ou thérapeutique selon l'invention est un animal, de préférence un mammifère, par exemple un rongeur, un canidé, un félin, un bovidé, un équidé, ou un primate. Un sujet est en particulier un humain, homme, femme ou enfant.

Le sujet présente une immunodépression post-traumatique. Généralement une immunodépression post-traumatique est consécutive à un ou plusieurs traumatismes sévères chez le sujet, tel qu'un traumatisme crânien.

Selon un mode de réalisation, le sujet traumatisé sévère en question présente au moins deux lésions traumatiques (polytraumatisme) parmi lesquelles au moins une lésion engage le pronostic vital. Le sujet est généralement hospitalisé, par exemple dans un service hospitalier de réanimation. Le sujet peu plus particulièrement être placé sous ventilation artificielle, notamment avec intubation.

Selon un mode de réalisation, le sujet traumatisé sévère présente un score de gravité des lésions (« Injury Severity Score » ou ISS) d'au moins 16. Pour les traumatismes très sévères, l'ISS est d'au moins 25. Le calcul du score ISS prend en compte l'atteinte de plusieurs régions de l'organisme du patient (tête et cou; face; thorax; abdomen et pelvis; bassins et membres; peau et tissus sous cutanés). L'atteinte de chaque région est cotée de 1 à 6 en fonction de sa gravité (1 : atteinte mineure, 6 : atteinte critique). L'ISS est ensuite calculé par la mise au carré du score des trois régions les plus atteintes (par exemple, si les côtes de chaque région sont les suivantes : tête 4, abdomen 3, thorax 2, autres régions 1, alors l'ISS vaudra 4²+3²+2² = 16+9+4 = 29 (Baker et al., 1974, J Trauma. 14 :187-196).

Selon un mode de réalisation, le sujet traumatisé sévère présente un traumatisme crânien sévère défini par un score de Glasgow (CGS) inférieur à 8. La détermination du CGS est une méthode qui permet d'apprécier la profondeur d'un coma par l'étude de la variabilité de 3 critères cliniques très précis qui sont : 1 ) l'ouverture des yeux (coté de 1 : absente à 4 : ouverture spontanée des yeux), 2) les capacités de motilité (faculté de se mouvoir), ou si l'on préfère la meilleure réponse motrice (cotée de 1 : absente à 6 : mouvements adaptés), et 3) la réponse aux questions posées (réponses verbales, cotée de 1 : absente à 5 : réponse orientée). Le CGS est la somme des résultats obtenus aux trois critères cliniques cités ci-dessus. Il est donc au minimum de 3 et au maximum de 15 (Teasdale et al. 1974, Lancet 2: 81-84).

L'immunodépression post-traumatique se caractérise de préférence par :
a) une baisse du niveau de production *ex vivo* de cytokines pro-inflammatoires induites par des leucocytes sanguins après stimulation par du LPS de bacilles Gram négatif, par exemple *Escherichia coli,* par rapport au niveau de production observé pour un individu sain ; et/ou
b) une baisse du niveau d'expression de HLA-DR sur les cellules présentatrices des antigènes du patient, par rapport au niveau d'expression observé pour un individu sain.

La diminution observée du niveau de production de cytokines (exprimé par exemple en picogrammes/ml) s'entend versus des volontaires sains dont le niveau de production de cytokines représente la valeur 100%. Les cytokines sont typiquement mesurées dans des cultures de sang total stimulées par du LPS (d*'Escherichia coli* en général). L'expression de HLA-DR s'exprime versus des volontaires sains, soit en nombre de molécules d'HLA-DR exprimées à la surface des cellules (MFI pour « mean fluorescence intensity »), soit en pourcentage, la valeur 100% représentant le niveau d'expression de HLA-DR chez les volontaires sains. De manière particulière, si l'on raisonne en pourcentage, la baisse en a) et/ou b) ci-dessus est d'au moins 20% environ. Elle est de préférence d'au moins 25% environ, de préférence encore d'au moins 30, 35, 40, 45% environ, et de manière plus préférentielle d'au moins 50, 55, 60% environ, voire plus.

De préférence encore, l'immunodépression post-traumatique observée chez le patient traumatisé sévère est telle que le niveau d'expression de HLA-DR sur les monocytes dudit dans les 24 heures suivant le ou les traumatismes (de J0 à J1) est diminué par rapport au niveau d'expression observé pour un individu sain. Cette diminution initiale peut d'ailleurs permettre, en pratique, de prédire le risque de complications septiques (ou infections secondaires). Ainsi, un bas niveau d'expression de HLA-DR sur les monocytes le premier jour suivant le ou les traumatismes sévères (J1), par exemple une diminution de 50%, est prédictif d'un risque élevé pour le patient de contracter une infection secondaire. L'expression de HLA-DR s'exprime versus des volontaires sains, soit en nombre de molécules d'HLA-DR exprimées à la surface des cellules (MFI pour « mean fluorescence intensity »), soit en pourcentage, la valeur 100% représentant le niveau d'expression de HLA-DR chez les volontaires sains. En particulier, si l'on raisonne en pourcentage, la diminution du niveau d'expression de HLA-DR est d'au moins 20% environ, de préférence d'au moins 25% environ, de préférence encore d'au moins 30, 35, 40, 45% environ, et de manière plus préférentielle d'au moins 50, 55, 60% environ, voire plus.

Selon un mode de réalisation la prévention et/ou le traitement de l'immunodépression post-traumatique conduit à la prévention et/ou traitement d'une complication septique de l'immunodépression post-traumatique.

Les complications septiques (ou infections secondaires) de l'immunodépression post-traumatique sont plus particulièrement des infections nosocomiales, notamment bactériennes ou fongiques ou virales. En particulier, les infections nosocomiales en cause sont sélectionnées dans le groupe constitué des pneumopathies, telles que les pneumonies acquises sous ventilation mécanique (PAVMs), des infections urinaires, des infections sur cathéters veineux centraux, des infections bactériennes cérébroméningées telles méningites empyèmes et abcès cérébraux. Plus particulièrement encore, les pneumopathies sont dues à des bactéries pathogènes choisies parmi les staphylocoques, de préférence *Staphylococcus aureus,* de préférence encore *Staphylococcus aureus* sensible à la méticilline, *Haemophilus sp.,* de préférence *H. influenzae,* les pneumocoques, les entérobactéries, *Pseudomonas sp.,* en particulier *P*. *aeruginosa.* Les bactéries à l'origine des pneumopathies peuvent également appartenir à d'autres genres ou espèces bactériens (par exemple, des bacilles Gram négatif et des coques Gram positif). De plus, elles peuvent être résistantes aux antibiotiques. S'agissant des infections autres que les pneumopathies, les bactéries ou levures responsables peuvent être, par exemple, des bacilles Gram négatif (e.g., *E. coli, Proteus mirabilis, Pseudomonas aeruginosa*) et des coques Gram positif (e.g., *S*. *aureus*) pour les infections urinaires ; des bacilles Gram négatif (e.g., *E. coli, P. mirabilis, P. aeruginosa*), des coques Gram positif (e.g., *S*. *aureus,* des staphylocoques coagulase négatif) et des levures telles que Candida sp. pour les infections sur cathéters centraux ; des bacilles Gram négatif (e.g., *E. coli, P. mirabilis, P. aeruginosa*) et des coques Gram positif (e.g., *S*. *aureus,* des staphylocoques coagulase négatif, *Streptococcus pneumoniae*) pour les infections bactériennes cérébroméningées.

Selon un mode de réalisation le lieu de l'infection (ou foyer d'infection) est le poumon.

L'extrait d'algues, ou la composition ou le médicament le contenant, est de préférence administré, une ou plusieurs fois si nécessaire, dans une période d'au plus 1 mois environ, de préférence d'au plus 28 jours, à compter de l'admission des patients à l'hôpital, en particulier dans un service hospitalier de réanimation. En d'autres termes, les complications septiques que l'on veut prévenir sont susceptibles de survenir (ou d'être contractées) pendant au plus 1 mois environ, de préférence au plus 28 jours, à compter de l'admission des patients à l'hôpital. Par exemple, on a pu constater que des complications septiques survenaient chez 40 à 50% des patients traumatisés sévères entre J0 et J10, et chez 10 à 20% des patients en plus entre J10 et J28, soit 50 à 70 % des patients au total avaient contracté une infection secondaire (Osborn et al., 2004 Crit. Care Med.;32(11):2234-40).

En particulier, l'extrait d'algues, ou la composition ou le médicament le contenant est administré une ou plusieurs fois pendant la période d'intubation des patients.

Dans la présente demande, le terme "et/ou" est une conjonction grammaticale qui doit être interprétée comme englobant le fait qu'un ou plusieurs des cas qu'elle connecte peuvent se produire. Par exemple, l'expression "du mannose et/ou de l'arabinose " dans l'expression "lesdits polysaccharides comprennent du mannose et/ou de l'arabinose " indique que les polysaccharides peuvent comprendre du mannose, ou de l'arabinose, ou du mannose et de l'arabinose.

Dans l'ensemble de la présente demande, le terme "comprenant" doit être interprété comme englobant toutes les caractéristiques spécifiquement mentionnées, ainsi que des caractéristiques facultatives, additionnelles, non spécifiées. Tel qu'utilisé ici, l'utilisation du terme "comprenant" décrit également le mode de réalisation dans lequel aucune caractéristique autre que les caractéristiques spécifiquement mentionnées n'est présente (c'est-à-dire "consistant en").

La présente invention sera illustrée plus en détail par les figures et exemples ci-dessous qui n'en limitent pas la portée.

### FIGURES

**Figure 1** **: Diagramme schématisant le protocole expérimental des analyses bactériologiques.**

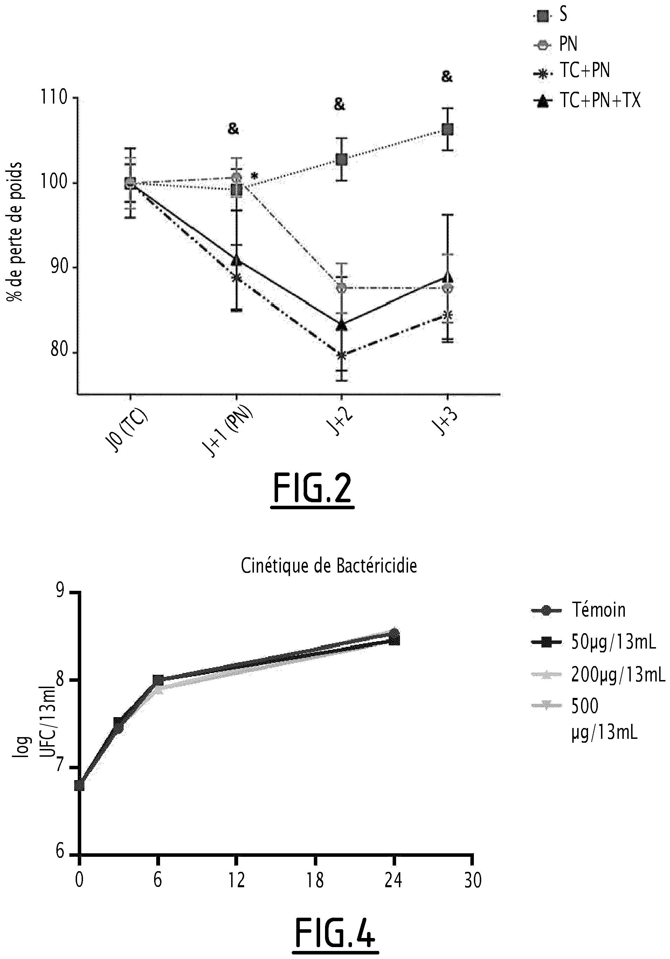

**Figure 2** **: L'extrait d'algues n'a pas d'effet sur la perte de poids des souris traumatisées et secondairement infectées.** Les souris du groupe Sham (S) et Pneumonie seule (PN) ont reçu l'incision de 1cm sur le crâne sans TC. Les souris traumatisées et infectées, pour lesquelles la pneumonie était réalisée 24h après le TC, étaient réparties dans le groupe non traité (TC+PN) et traité par 6 injections du composé (toutes les 12h du TC jusqu'à l'euthanasie) (TC+PN+TX). La pneumonie était réalisée 24h après le TC et les souris euthanasiées 48h après la pneumonie. Les résultats sont issus de 2 expériences indépendantes (chaque groupe n= 9). Les résultats de % de perte de poids sont donnés en moyennes ± écart type. * p<0,05 groupes traumatisés et PN (après la PN) versus S, * p<0,05 pour les groupes traumatisés versus PN.

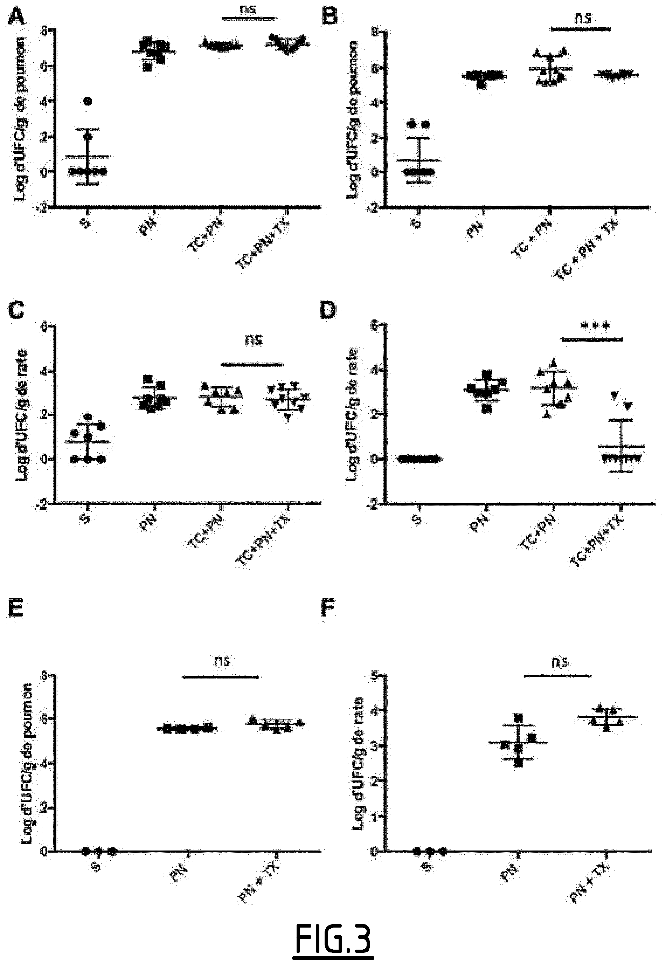

**Figure 3** **: L'extrait d'algues limite la dissémination bactérienne splénique chez les souris traumatisées après l'induction d'une pneumonie à SAMS mais n'a aucun effet en absence de traumatisme** : Les souris du groupe Sham **(S)** et Pneumonie seule (PN) ont reçu l'incision de 1cm sur le crâne sans TC. Les souris traumatisées et infectées étaient réparties dans le groupe non traité **(TC+PN)** et traité par le composé (200µg toutes les 12h du TC jusqu'à l'euthanasie) **(TC+PN+TX).** Les poumons ont été prélevées 24h **(A)** et 48h **(B)** après l'induction de la pneumonie. Les rates ont été prélevées 24h **(C)** et 48h **(D)** après l'induction de la pneumonie. Les organes broyés ont été incubé sur géloses Chapman pendant 24h puis les colonies ont été dénombrées. Les résultats sont issus de 2 expériences indépendantes (n=8 ou 9 par groupes) et sont donnée en moyenne de Log₁₀ d'UFC ± écart -type. ***p<0,001. **(E)** et **(F)** : Les souris étaient réparties en 3 groupes : Sham **(S),** pneumonie non traitée **(PN)** et pneumonie traitée par l'extrait d'algues toutes les 12 heures pendant 48 heures (sans traumatisme) **(PN+TX).** Les poumons **(E)** et les rates **(F)** ont été prélevés 48h après l'induction de la pneumonie. Les résultats sont issus d'une expérience (n=5 par groupe d'intérêt). Les résultats sont donnés en moyenne ± écart type.
**Figure 4** **: L'extrait d'algues ne possède pas d'activité anti-SAMS *in vitro.*** La cinétique de bactéricidie a été réalisée en milieu liquide pour des gammes de concentration de 50, 200 et 500 µg/mL du composé. Les charges bactériennes ont ensuite été dénombrées sur géloses TS et les résultats exprimés en Log₁₀ d'UFC/mL. Les résultats sont issus de 2 expériences indépendantes et concordantes.

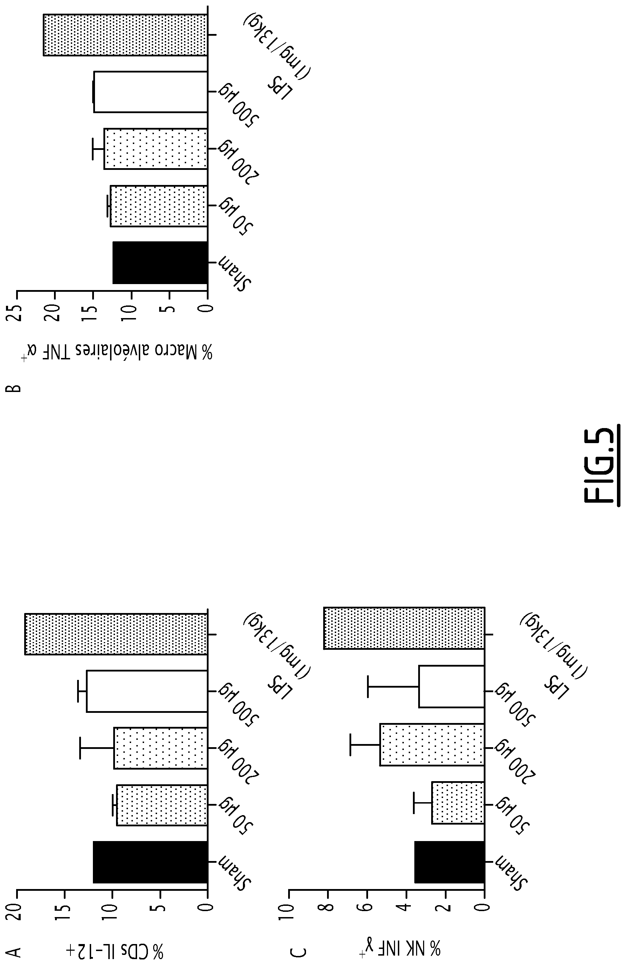

**Figure 5** **: L'administration intrapéritonéale de l'extrait d'algues n'induit pas d'augmentation de la sécrétion de cytokines pro-inflammatoire à 2 heures chez la souris naïve.** L'injection intrapéritonéale de l'extrait d'algues était réalisée à 3 concentrations (50, 200 et 500 µg) et était comparée à l'injection de PBS (Sham) et à 1mg/kg de LPS. Les sécrétions d'IL-12 par les CDs pulmonaires **(Fig 5A),** de TNFα par les macrophages alvéolaires **(Fig 5B)** et interstitiels ainsi que d'INF γ par les NK **(Fig 5C)** et LT pulmonaires étaient évaluées en cytométrie en flux après marquage intracellulaire. Aucune différence n'était retrouvée dans la rate (données non montrées). Les résultats sont issus d'une expérience (n=2 par groupe) et sont donnés en médiane ± intervalles interquartiles.

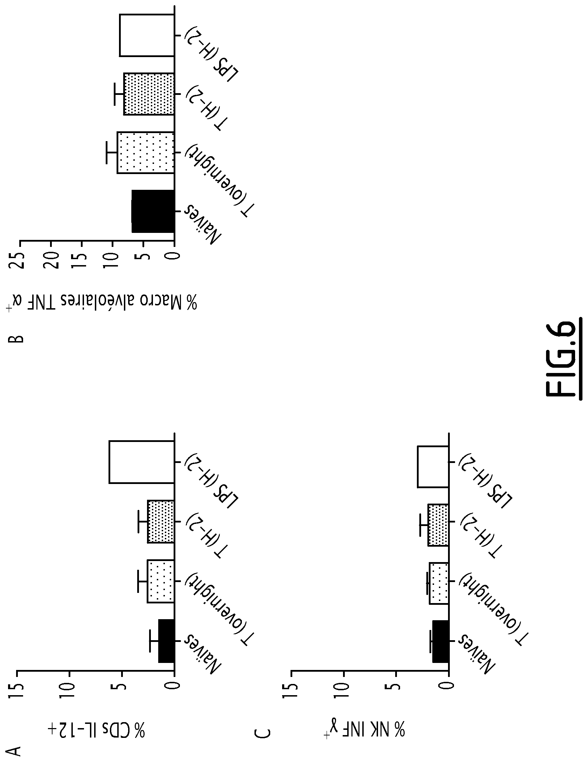

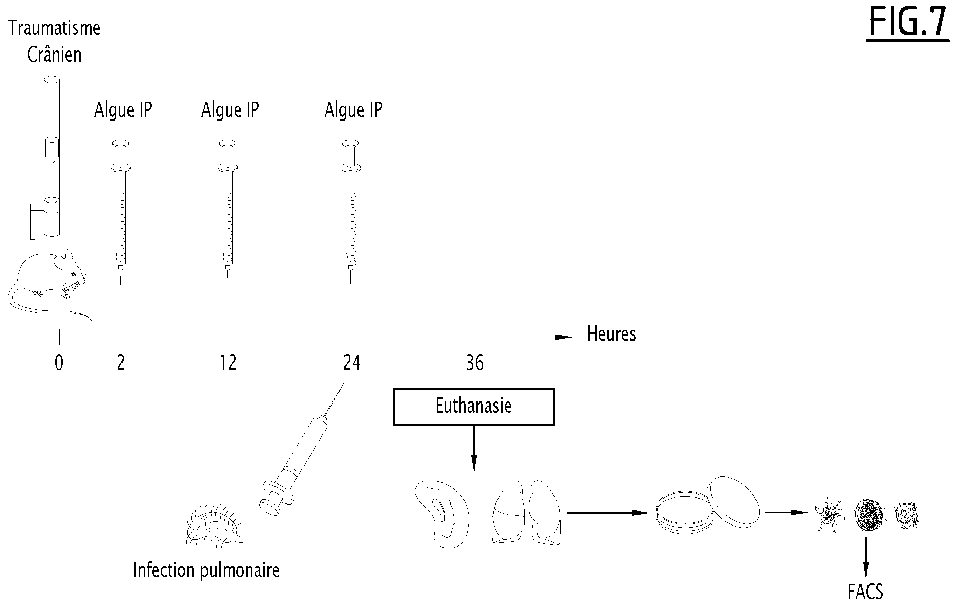

**Figure 6** **: L'administration intratrachéale de l'extrait d'algues n'induit pas d'augmentation de la sécrétion de cytokines pro-inflammatoire à 2 heures et à 12 heures chez la souris naïve.** L'injection intratrachéale de l'extrait d'algues était réalisée à la concentration de 50µg 12 heures **(T overnight)** et 2 heures (T **(H-2))** avant l'euthanasie. Elle était comparée à des souris saines **(Naïves)** et à l'instillation de 50µg de LPS deux heures avant l'euthanasie **(LPS (H-2)).** Les sécrétions d'IL-12 par les CDs pulmonaires **(Fig 7A),** de TNFα par les macrophages alvéolaires **(Fig 7B)** et interstitiels ainsi que d'INF γ par les NK **(Fig 7C)** et LT pulmonaires étaient évaluées en cytométrie en flux après marquage intracellulaire. Aucune différence n'était retrouvée dans la rate (résultats non montrés). Les résultats sont issus d'une expérience (n=2 par groupe) et sont donnés en médiane ± intervalles interquartiles.
**Figure 7** **: Diagramme schématisant le protocole expérimental des analyses en FACS et ELISA.**

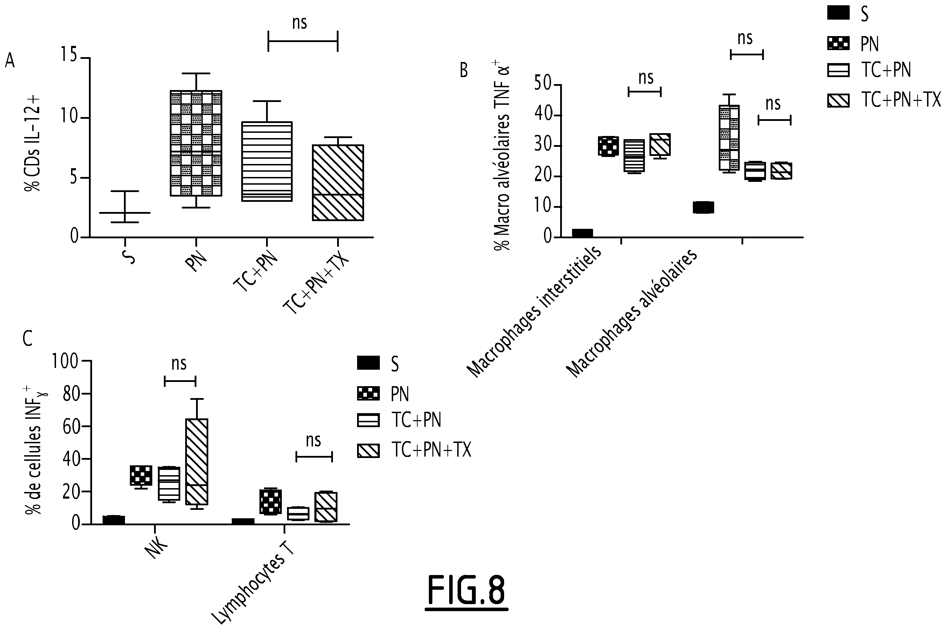

**Figure 8** **: L'administration intra-péritonéale de l'extrait d'algues chez les souris traumatisées souffrant d'une pneumonie à SAMS n'augmente pas la proportion de cellules produisant des cytokines pro-inflammatoires dans le poumon.** Les souris étaient réparties en 4 groupes : Sham **(S),** pneumonie seule **(PN),** TC + pneumonie non traitées **(TC+PN)** et traitées par l'extrait d'algues **(TC+PN+TX).** La pneumonie était instillée 24 heures après le TC et les souris sacrifiées 12 post pneumonie. Les souris du groupe traité recevaient 3 injections intrapéritonéale de 200 µg de l'extrait d'algues (toutes les 12 heures, du TC au sacrifice). Les sécrétions d'IL-12 par le CDs **(A),** de TNFa par les macrophages **(B)** et d'INF γ par les LT et NK **(C)** étaient analysées en cytométrie en flux après marquage intracellulaire. Les résultats sont issus de 2 expériences différentes (n=4 par groupe). Les résultats sont donnés en médiane de pourcentage de cellules positives ± intervalles interquartiles. Aucune différence n'était retrouvée dans la rate (résultats non montrés).

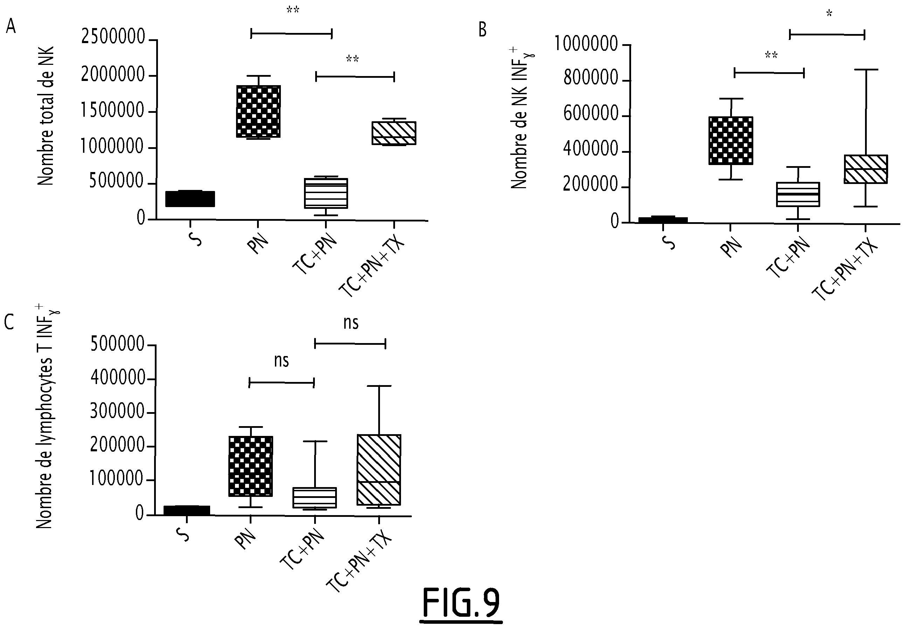

**Figure 9** **: L'administration de l'extrait d'algues chez la souris traumatisée infectée induit une augmentation du nombre de cellules NK pulmonaires productrices d'interféron γ.** Les souris étaient réparties en 4 groupes : Sham **(S),** pneumonie seule **(PN),** TC + pneumonie non traitée **(TC+PN)** et traitée par l'extrait d'algues **(TC+PN+TX).** La pneumonie était instillée 24 heures après le TC et les souris sacrifiées 12 heures post pneumonie. Les souris du groupe traité recevaient 3 injections intrapéritonéale de 200 µg de l'extrait d'algues (toutes les 12 heures, du TC jusqu'au sacrifice). Le nombre total de cellules NK **(A),** de NK produisant de l'interféron γ **(B)** et de LT produisant de l'interféron γ **(C)** ont été analysés par cytométrie en flux après marquage intracellulaire des cytokines. Les résultats sont issus de 3 expériences indépendantes (n=9 par groupe). Les résultats sont donnés en médiane ± intervalles interquartiles. (*p<0,05, **p<0,01).

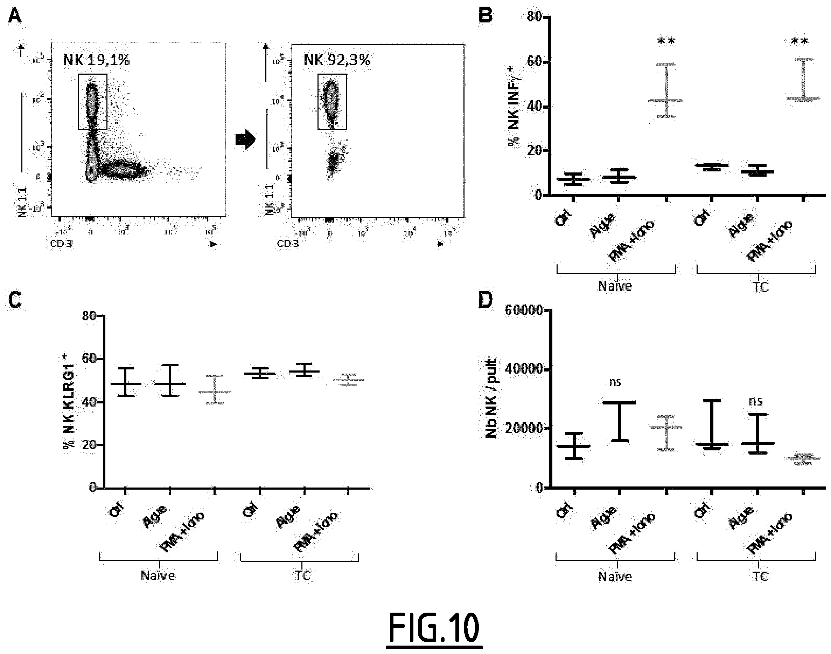

**Figure 10** **: L'extrait d'algues n'induit pas d'augmentation de la sécrétion d'INF γ ni d'augmentation d'expression du facteur d'activation KLRG 1 par les cellules NK pulmonaires *in vitro* :** Les poumons de 6 souris naïves et de 6 souris traumatisées ont été broyés mécaniquement deux par deux 24h après le TC afin d'obtenir 3 homogénats pulmonaires par groupes. Après avoir été triées magnétiquement, les cellules NK ont été mises 5 heures en cultures avec de l'IL-2 (groupe contrôle : **Ctrl**), de l'IL-2 + Algue à 500 µg/mL **(Algue)** et de l'IL-2 + PMA (50ng/mL) et lonomycine (1µg/mL) **(PMA + lono).** La pureté de l'homogénats en cellules NK a été contrôlée en cytométrie en flux avant (**A** gauche) et après (**A** droite) tri magnétique. Le pourcentage de NK produisant de l'INF γ **(B),** l'expression membranaire de KLRG 1 **(C)** et le nombre de NK par puit après stimulation **(D)** ont été évalués en cytométrie de flux. Les résultats sont issus d'une expérience (n=3 par groupe) et donnés en médiane ± intervalles interquartiles. **p<0,01 comparé aux groupes Ctrl et Algue. « Ns » comparé aux 2 autres groupes.

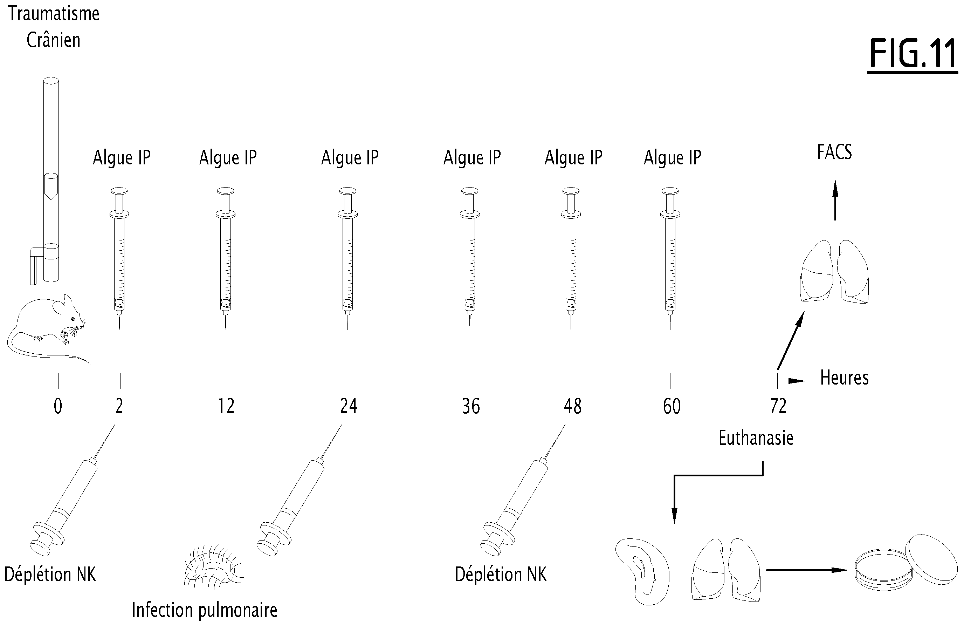

**Figure 11** **: Diagramme schématisant le protocole expérimental des analyses en FACS et en bactériologie.**

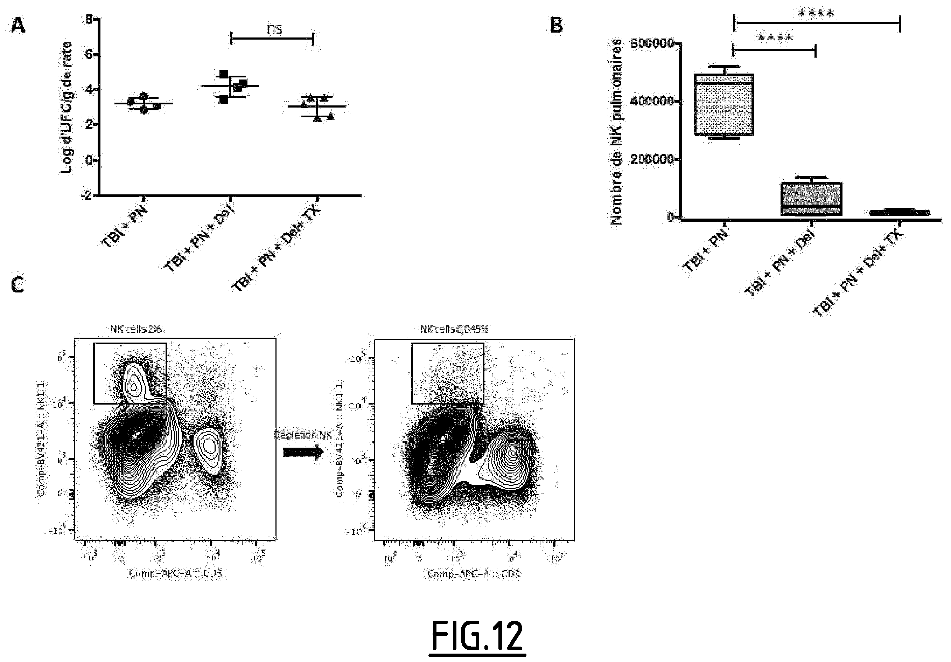

**Figure 12** **: L'administration de l'extrait d'algues chez la souris traumatisée déplétée en cellules NK ne limite pas la dissémination bactérienne splénique à 48h de l'infection.** Les souris étaient réparties en 3 groupes : traumatisées et infectées **(TBI + PN),** traumatisées, infectées et déplétées en cellules NK **(TBI + PN+ del),** traumatisées, infectées, déplétées en cellules NK et traitées par l'extrait d'algues **(TBI + PN + del + TX)** La pneumonie était instillée 24 heures après le TC et la déplétion de NK était réalisée par injection IV d'Ac anti NK1.1 à 2h et 48h après le TC. Les souris du groupe traité recevaient 1 injection intrapéritonéale de 200 µg de l'extrait d'algues toutes les 12 heures, du TC au sacrifice. L'euthanasie était réalisée à 72h du TC pour analyse des charges bactérienne splénique **(****Fig 12 A)** et de la qualité de la déplétion des NK pulmonaires en FACS **(****Fig 12B et C).** Les résultats sont issus d'une expérience (n=4 par groupe) et donnés en médiane ± intervalles interquartiles. ****p<0,0001.

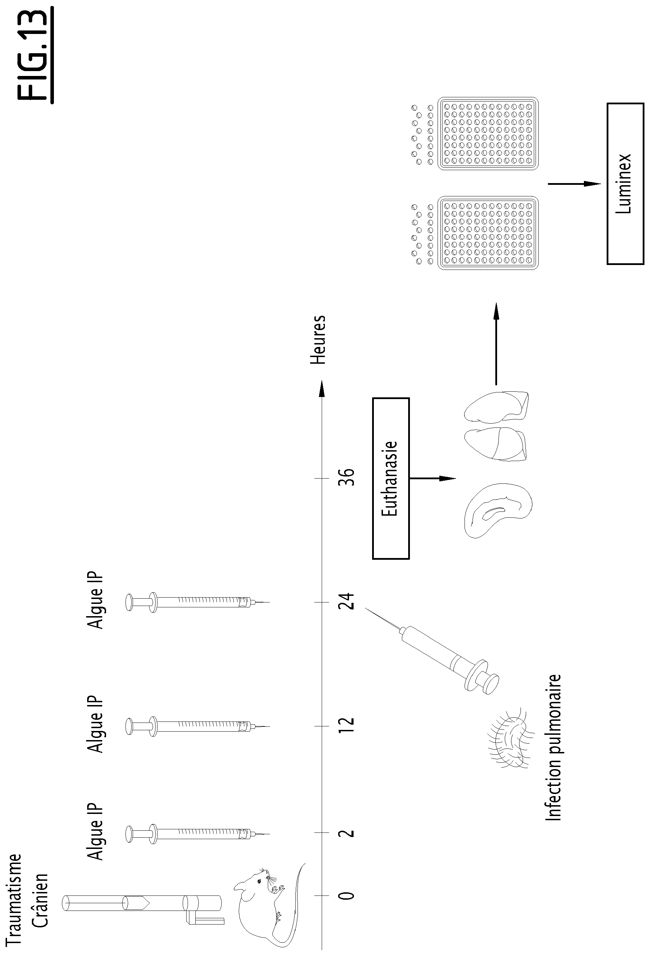

**Figure 13** **: Diagramme schématisant le protocole expérimental des analyses des chimiokines spléniques et pulmonaires par Luminex.**

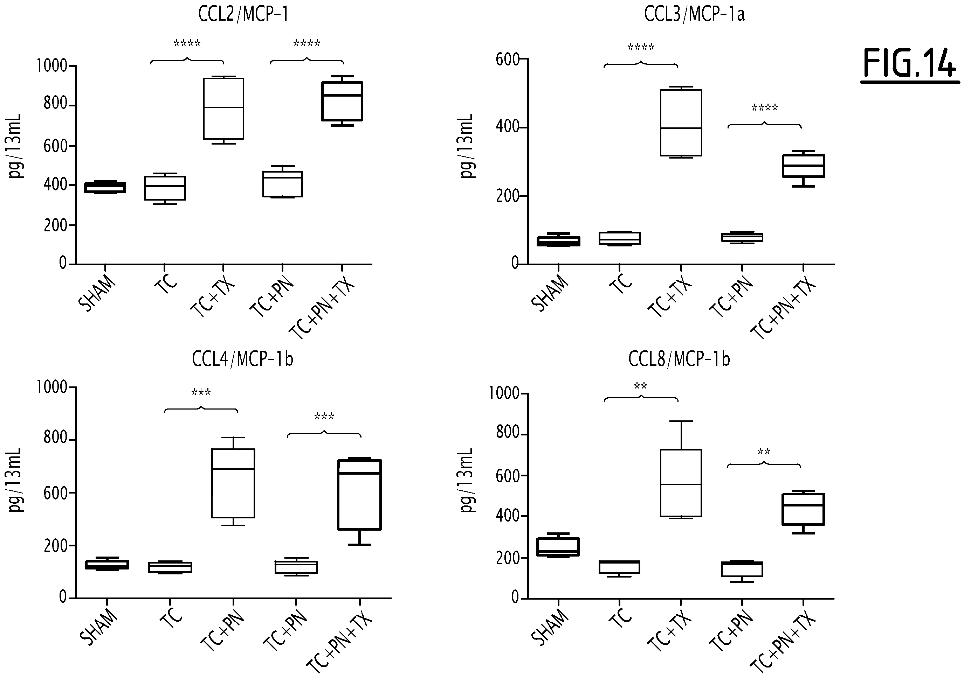

**Figure 14** **: L'administration du polysaccharide chez la souris traumatisée seules et traumatisée/infectée induit une augmentation de la concentration splénique des chimiokines CCL2, CCL3, CCL4 et CCL8.** Les souris étaient réparties en 5 groupes : Naïves (SHAM), traumatisme seul (TC), TC + traitement par le composé(TC+TX) traumatisme + pneumonie (TC+PN) et traumatisme + pneumonie + traitement par le composé (TC+PN+TX). La pneumonie était provoquée 24 heures après le TC et les souris sacrifiées 12 après la pneumonie. Les souris des groupes traités recevaient 3 injections intrapéritonéale de 200 µg du composé marin (toutes les 12 heures, du TC au sacrifice). Les rates étaient prélevées 12 heures après l'induction de la pneumonie, broyés puis le taux de chimiokines CCL2 **(A)** et CCL3 **(B)** CCL4 **(C)** et CCL8 **(D)** ont été mesuré par technique Luminex. Les résultats sont issus d'une expérience (n=5 souris par groupe). Les résultats sont donnés en médiane +/- intervalles interquartiles ***p<0,001, ****p<0,0001.

### EXEMPLES

### Exemple 1 : Préparation d'un extrait d'algues pour l'utilisation selon l'invention

L'extrait d'algues est préparé comme décrit dans l'exemple 1 de la demande de brevet internationale WO2015071497.

Une tonne d'algues vertes de type *Ulva,* fraîches, brutes, sont lavées à l'eau douce et dessablées à l'aide d'une machine à laver les algues.

Sauf indications contraires, les étapes du procédé sont réalisées à température ambiante.

Les algues (1 tonne d'algues égouttées à 8% de matière sèche) sont ensuite broyées en fines particules au moyen d'un affineur industriel (marque Inotec type "I175CDI-75D"). Par « fines particules », on entend des particules dont la taille est comprise entre 50 et 1000 nm, avec deux populations, la première dont les tailles sont comprises entre 50 et 200 nm, la seconde dont les tailles sont comprises entre 600 et 1000 nm.

Le broyat est ensuite pressé au moyen d'une presse à bande industrielle de marque Flottweg type "B FRU 800 HK" à un débit d'environ 1 tonne/heure. Cette étape permet la séparation de la phase solide (marc) de la phase liquide (jus). Le rendement en jus obtenu est de 75%.

Les 750 kg de jus brut obtenus sont ensuite clarifiés au moyen d'un clarificateur à assiettes de marque Flottweg type "AC 2000". On obtient ainsi 710 kg d'un jus clair à 3.10 % de masse sèche (95 à 98% de rendement en masse) et une crème (2 à 5% en masse).

Par la suite, le jus clair est ultrafiltré sur une membrane céramique (Tami Industries) de 15 KDa. On obtient ainsi un perméat et un rétentat. Le perméat est conservé jusqu'à l'obtention de 640 kg de jus de filtration (91% de rendement en volume) à 2.2% de matière sèche.

Le jus de filtration (perméat) est alors séché par lyophilisation après concentration par évaporation.

La concentration est réalisée sur un évaporateur simple effet (EVA 1000, Pignat) avec les paramètres suivants: recirculation forcée, débit d'alimentation 10L/h, pression vapeur de 1 bar, pression de vide de 0,3 bar et température d'évaporation de 90°C. Une première concentration est réalisée avec un débit d'eau évaporée de 8 L/h et le °brix monte de 5.5 (égal à une concentration de matière sèche de 4.5%) à 14.7. Cette solution est ensuite concentrée une deuxième fois avec un débit d'eau évaporée de 5-6 L/h et le °brix monte jusqu'à 34. La concentration de matière sèche de la solution est déterminée à 38.4%.

La lyophilisation est ensuite réalisée à l'aide d'un appareil Bioblock scientific (modèle CHRIST alpha 1-4 LSC) à une température de congélation de -80°C qui est également la température minimale au cours de cette étape.

La poudre obtenue est ensuite broyée avec un broyeur planétaire MiniMill de marque Philips. Le produit a été introduit dans des bols de broyage (10 g de produit dans chaque bol de broyage avec 4 billes en zircone). L'ensemble a été mis en rotation pendant 15 minutes à la vitesse 10. On obtient ainsi 14 kg de poudre d'extrait d'algues.

### Exemple 2 : Etude de l'effet de l'extrait d'algues dans un modèle murin de traumatisme crânien - Matériels et méthodes

Bien-être animal : Toutes les expérimentations ont été menées en accord avec les principes du bien-être animal en laboratoire. Toutes les expériences ont été approuvées par le comité d'éthique des Pays de la Loire et par le MESR (n°2016121915529061). Les souris (Swiss mâles de 5 semaines, souche RjOrl : SWISS et C57BL/6 mâles 6 semaines, souche C57BL/6JRj de 24 à 28 g) ont été obtenues auprès du laboratoire Janvier (Laval). Les souris ont été maintenues dans un cycles jour/nuit de 12h avec accès libre à l'eau et à la nourriture à l'animalerie de l'Institut de Recherche en Santé 2 (IRS 2) de Nantes, France.

L'extrait d'algues comprenant des polysaccharides sulfatés et non sulfaté: Il a été extrait et purifié à partir de l'algue *Ulva Armoricana* collectée sur la plage à Plestin les Grèves (Bretagne, France), selon le protocole décrit dans l'exemple 1. L'absence de contamination des différentes fractions par le LPS a été évaluée à l'aide d'une technique disponible dans le commerce (E-toxate Kit, Sigma). Aucun LPS n'a été détecté par ce dosage dans l'extrait final. Les échantillons utilisés étaient sous forme de solution aqueuse à la concentration de 1% de polysaccharides sulfatés et non sulfatés (concentration en poids de la matière sèche par rapport au volume final de la solution).

Modèle de traumatisme crânien : Le traumatisme crânien a été réalisé par la technique du « weight drop device » (Flierl et al., 2009, Nat. Protoc.;4(9):1328-37). Une injection sous-cutanée de 0,1 mg/kg de buprénorphine était réalisée trente minutes avant la procédure puis les souris étaient anesthésiées par inhalation continue d'isoflurane (débit de gaz frais 0,8L/min, fraction inhalée 3,5%). Une incision de 1 cm au sommet du crâne permettait de repérer les sutures coronales et sagittales et d'optimiser la procédure. Le traumatisme était alors réalisé par la chute d'un poids standardisé de 2,5 cm de hauteur, puis l'incision était refermée par un point au fil 4.0. Il ne devait pas y avoir d'effraction de la boîte crânienne. La récupération *ad integrum* était surveillée immédiatement puis toutes les 12 heures et les souris bénéficiaient d'une analgésie par buprénorphine sous-cutanée si nécessaire. En cas de réveil pathologique, les souris étaient euthanasiées. Les points limites étaient évalués selon le tableau I ci-dessous :

**Tableau I : Points limites. Les souris étaient euthanasiées pour un score supérieur ou égal à 6.**

| | **0 point** | **1 point** | **2 points** | **3 points** |
|---|---|---|---|---|
| **Perte de poids** | <5% | 5-12% | 13-20% | >20% |
| **Apparence physique (poils hérissés et dos bossu)** | Normale | Peu altérée | Modérément altérée | Très altérée |
| **Comportement (isolement)** | Normal | Peu modifié | Modérément modifié | Très modifié (isolement permanent du reste du groupe) |

L'inoculum bactérien : La souche de SAMS ATCC 29213 (hémolysine positive, leucocidine de Panton Valentine négative) a été utilisée pour toutes les expériences. La souche était mise en incubation en bouillon coeur-cervelle pendant 18h à 37°C puis était lavée 2 fois (1000g pendant 10 min à 20°), et enfin diluée dans du PBS stérile. Ensuite, l'inoculum était calibré par néphélométrie afin d'obtenir 7 MacFarland puis finalement concentré 5 fois afin d'atteindre une concentration entre 1 et 3 ×10⁹ UFC/mL.

Modèle de pneumonie : Une injection sous-cutanée de 0,1 mg/kg de buprénorphine était réalisée trente minutes avant la procédure puis les souris étaient anesthésiées par inhalation continue d'isoflurane (débit de gaz frais 0,8 L/min, fraction inhalée 3,5%). La pneumonie était ensuite induite par l'insertion intra-trachéale d'une canule de gavage de 24 gauges puis par l'injection de 75 µL d'inoculum. Les souris étaient réparties en 4 groupes : Les souris Sham **(S)** non infectée non traumatisées, les souris infectées non traumatisées **(PN),** les souris traumatisées et infectées, non traitées **(PN+TC)** et les souris traumatisées infectées et traitées par 200µg du composé marin en intra-péritonéal toutes les 12 heures **(PN+TC+TX).**

L'administration de l'extrait d'algues : L'extrait d'algues était administré aux doses de 50 µg, 200 µg et 500 µg dans un volume total de 200 µL (complété par du PBS) par voie intrapéritonéale ou intratrachéale.

Recherche d'un effet dose de l'extrait : L'extrait d'algues a été administré par voie intrapéritonéale 2 heures et 12 heures avant l'euthanasie aux doses de 50, 200 et 500 µL dans un volume total de 200µL de PBS. Afin de stimuler *in situ* les cellules de l'immunité muqueuse pulmonaire, l'extrait d'algues a également été administré à la dose de 50 µg dans 75 µL par voie intra-trachéale selon la même procédure que pour l'inoculation bactérienne lors de la pneumonie. Les rates et poumons ont ensuite été prélevés puis les populations cellulaires analysées en cytométrie en flux après marquages intra-cellulaires des cytokines pro-inflammatoires (IL-12, TNFα, INF γ).

Analyse par FACS des populations cellulaires : Les suspensions cellulaires pulmonaires et spléniques ont été obtenues par broyage mécanique manuel puis digestion par collagénase durant 30 minutes (rates) ou 45 minutes (poumons) puis passées au tamis (pores de 70µm). Après traitement par une solution de lyse des hématies, les suspensions cellulaires obtenues étaient incubées 30 minutes à 4°C avec les anticorps couplés aux fluorochromes spécifiques.

Pour les CDs : CD24-BV711 (M1/69, BD Horizon), CD11c-PeCy7 (NK18, Biolegende), MHC II-BV421(M5/114.15.2, Biolegend) ; Pour les macrophages : F4/80-Alexa647(BM8, Biolegende), CD11b-BV605 (M1/70, BD Horizon), CD11c-PeCy7(NK18, Biolegend). Pour les cellules NK et lymphocytes T : NK1.1-BV421(PK136, BD Horizon), CD3-PE (145-2C11, eBioscience) et KLRG 1-APC (2F1, eBioscience). Les cellules ont été analysées sur l'appareil BD LSR II^{®} puis l'ensemble des données ont ensuite été interprétées à l'aide du logiciel Flowjo Software^{®} (TreeStar Inc, Ashland, OR).

Le marquage des cytokines intra-cellulaires : Les souris ont été euthanasiées 12 heures après l'instillation trachéale de SASM créant la pneumonie. Pour le marquage intracellulaire des cytokines dans les DCs, les macrophages et les lymphocytes, les cellules ont été incubées 5 heures dans un milieu comprenant du RPMI et du *Golgi Plugh* (BD Bioscience) afin de bloquer l'exocytose, lavées deux fois puis marquées selon les marqueurs membranaires (cf ci-dessus). La fixation et la perméabilisation ont été effectuées selon les recommandations du fabricant (BD Cytofix/Cytoperm kit, BD Bioscience). Les cellules ont été incubées pendant une nuit à 4°C dans du PermWash avec les anticorps anti IL12-PE (C15.6, BD Pharmingen), INF γ-Alexa488 (XMG1.2, eBioscience) et TNFα-PE (MP6-XT22, BD Pharmingen) puis les cellules ont été lavées deux fois et analysées par cytométrie en flux.

L'évaluation de la croissance et de la dissémination bactérienne : Les poumons et les rates ont été pesés puis broyés mécaniquement dans des conditions stériles. Les homogénats d'organes ont été soumis à plusieurs dilutions (de 10⁻² à 10⁻⁶ selon les conditions) et ont été incubés à 37 ° C sur un milieu spécifique (Chapman) afin d'éviter la croissance d'autres bactéries. Après une incubation de 24h, les colonies ont été dénombrées et les résultats ont été exprimés en log₁₀ d'UFC par gramme d'organe.

La cinétique de bactéricidie : la cinétique de bactéricidie en milieu liquide a été réalisé à partir d'un inoculum de SAMS ATCC 29213 à 0,5 de MacFarland (données néphélométriques) dilué 5 fois dans un bouillon Mueller Hinton (inoculum de départ =6×10⁶ UFC/mL). Le témoin a été comparé à plusieurs gammes de concentrations du composé sulfaté marin (50 µg/mL, 200 µg/mL, 500 µg/mL). 50 µL ont ensuite été ensemencés à H0, H3, H6 et H24 sur gélose TS puis incubé à 37° pendant 24h. Les colonies ont été dénombrées et les résultats exprimés en log₁₀ d'UFC/mL.

Détermination du niveau d'expression des chimiokines pulmonaires et spléniques par méthode Luminex. Après prélèvements des poumons et des rates, ces derniers ont été homogénéisés mécaniquement à 4°C en présence de tampon de lyse (1xPBS, pH 7,4/ 0,1% triton X-100) contentant 1mM de protease inhibitor cocktail (Sigma, l'Isle D'Abeau Chesnes, France). Les homogénats ont ensuite été centrifugés à 12 000g pendant 20 minutes à 4° puis le surnageant a été prélevé puis stocké à -80°C jusqu'à l'analyse. Les concentrations des différentes chimiokines ont été réalisé par la plateforme CIMNA (Centre D'Immunomonitorage Nantes Atlantique) par méthode Luminex ^{®} après marque spécifiques des différentes chimiokines : CCL19/MIP-3 beta (BR19), CCL2/MCP-1/JE (BR18), CCL20/MIP-3 alpha (BR48), CCL21/6Ckine (BR72), CCL3/MIP-1 alpha (BR46), CCL4/MIP-1 beta (BR51), CCL8/MCP-2 (BR38), CXCL1/KC (BR13), CXCL10/IP-10 (BR37), CXCL2/MIP-2 (BR20).

Tri magnétique des cellules NK : Les suspensions cellulaires pulmonaires ont été obtenues selon le protocole utilisé pour l'analyse FACS. Les cellules ont été comptées (environ 2.10⁷ par poumon) puis centrifugées à 300g pendant 10 minutes. Les cellules ont été suspendues dans 40µL de FACS Buffer pour 10⁷ cellules puis marquées par 10µL de NK Cell Biotin- Antibody Cocktail (*Miltenyi* Biotec^{®}, Germany) et incubée 5 minutes à 4°C. Après une nouvelle centrifugation à 300g pendant 10 minutes, les cellules ont été incubées 10 minutes à 4°C avec 20 µL d'Anti-Biotin MicroBeads (*Miltenyi* Biotec^{®}, Germany) pour 10⁷ cellules. Enfin la séparation magnétique a été réalisée selon les recommandations du fabricant (Miltenyi Biotec, Germany). Chaque groupe (naïve et TC) était composé de 6 souris puis les poumons ont été broyés deux par deux afin d'atteindre au minimum 10 000 cellules NK par puit (pour un total d'environ 20 millions de cellules par poumons de souris). La pureté en cellules NK était supérieure à 90%.

Stimulation *in vitro* des cellules NK : Les cellules NK isolées après tri magnétique ont été stimulées *in vitro* durant 5 heures dans l'étuve en CO₂ à 37°C et selon 3 conditions différentes : Contrôle (RPMI + SVF + IL-2 à la concentration de 3000 UI/mL), Algues (contrôle + Algue à 500µg/mL) et PMA-lonomycine (contrôle + PMA à 50ng/mL et lonomycine (1µg/mL). Les cellules étaient également stimulées 4 heures sur les 5 avec *Golgi Plugh* (BD Bioscience) selon les recommandations du fabricant. La sécrétion d'IFN-g, l'expression membranaire de KLRG 1 et le nombre de cellules NK ont ensuite été analysées par FACS.

Déplétion *in vivo* des cellules NK : Les cellules NK était déplétées in vivo par injections IV (rétro-orbitaires) de 10µL de LEAF purified anti mouse NK1.1 (clone PK136/ cat# 108712 - 1mg/mL) (Biolegend) dans 190 µL de PBS. Les injections étaient réalisées 2h et 48h après le traumatisme crânien. L'efficacité de la déplétion de NK était évaluée en FACS par le compte du nombre de cellules NK pulmonaires : NK1.1-BV421(PK136, BD Horizon), CD3-PE (145-2C11, eBioscience).

Statistiques : Les analyses statistiques ont été réalisées avec le logiciel GraphPadPrism 6.0^{®} (San Diego, CA, United States). Les variables continues non paramétriques ont été exprimées en médianes +/- écarts interquartiles et analysées par un test de Kruskall-Wallis avec utilisation post-hoc du test de Dunn. Les analyses des populations en pourcentage ont été réalisées par un test de Fisher bilatéral.

### Exemple 3 : Effets cliniques et bactériologiques

Les souris étaient réparties en 4 groupes : Sham **(S),** Pneumonie seule **(PN),** Traumatisme crânien + Pneumonie **(TC+PN),** et les souris traumatisées et infectées, traitées toutes les 12 h par injection intra-péritonéale (IP) de 200µg du composé **(TC+PN+TX)** en commençant 2h après traumatisme et jusqu'à l'euthanasie. Les souris des groupes S et PN recevaient une incision de 1 cm du haut du crâne sans le traumatisme. La pneumonie était induite 24 heures après le traumatisme crânien. L'euthanasie survenait 24h à 48h après l'induction de la pneumonie selon les critères étudiés **(****Figure 1****).**

### L'extrait d'algues [n'a pas d'effet sur la perte de poids des souris traumatisées et secondairement infectées

La pneumonie à SASM induit une perte de poids transitoire atteignant environ 12% du poids du corps à 24h heures. Le traumatisme crânien augmente la perte de poids à la phase initiale de l'infection mais ne modifie pas la récupération à partir de J+3 comparé aux souris non traumatisées. Le traitement n'a pas d'effet sur la perte de poids des souris traumatisées et secondairement infectées **(****Figure 2****).**

### L'extrait d'algues limite la dissémination bactérienne chez les souris traumatisées 48h après l'induction d'une pneumonie à SAMS

Les charges bactériennes pulmonaires sont similaires dans tous les groupes infectés à 24h (PN, TC+PN, TC+PN+TX) **(****Figure 3A****)** ainsi qu'à 48h **(****Figure 3B****)** de l'induction de la pneumonie.

Dès la 24^{ème} heure suivant l'infection pulmonaire, toutes les souris ayant subi une pneumonie étaient bactériémiques à SAMS (charge bactérienne splénique> 2 log).

Les souris traumatisées ne présentent pas de dissémination bactérienne supérieure après une pneumonie comparée aux souris non traumatisées (PN vs TC+PN) **(****Figure 3C et 3D****).**

Le traitement n'a aucun effet sur la bactériémie à 24h de l'infection **(****Figure 3C****)** mais à la 48^{ème} heure de l'infection, le *Staphyloccocus aureus* était mis en évidence chez 22% des souris du groupe traité (TC+PN+TX) contre 100% dans le groupe subissant la pneumonie post-traumatique non traité (TC+PN). Les souris du groupe traité par l'extrait d'algues présentent donc des charges bactériennes spléniques inférieures à 48h de l'induction de la pneumonie **(****Fig 3D****).** Cet effet semble être en relation avec la fréquence d'administration car l'injection unique de l'extrait d'algues induit une diminution de la bactériémie inférieure à celle observée en cas d'injections multiples (résultats non montrés).

Bien qu'il n'existe pas de différence de charges bactériennes entre le groupe pneumonie seule (PN) et le groupe traumatisé infecté (TC+PN) **(****Figure 3C et 3D****),** le traitement adjuvant (injecté avant l'infection) diminue la dissémination bactérienne au cours des infections post-traumatiques **(****Figure 3D****),** mais pas en l'absence de traumatisme **(****Figure 3E****).** Ce résultat suggère que l'extrait d'algues améliore la réponse pulmonaire à une infection bactérienne en cas d'immunodépression post-traumatique, mais n'a pas ou peu d'effet au cours d'une pneumonie sur animaux sains.

### L'extrait d'algues n'a pas d'activité spécifique anti-SAMS in-vitro

Au cours d'une pneumonie post-traumatique, le traitement diminue la durée de la dissémination systémique de l'infection bactérienne **(****Figure 3****).** Cet effet thérapeutique pourrait être lié soit à un effet antibactérien direct de l'extrait d'algues, soit à un effet sur l'immunité de l'hôte. Pour étudier les éventuelles propriétés antibactériennes directes, nous avons évalué l'effet de l'extrait d'algues sur la souche de SAMS ATCC 29213. Pour cela nous avons évalué *in vitro* les effets de l'extrait d'algues sur la croissance du SAMS par la méthode des cinétiques de bactéricidie en milieu liquide. Les résultats montrent que de l'extrait d'algues n'a aucun effet anti-SAMS direct quelle que soit la concentration testée, y compris à des doses supra-thérapeutiques (50, 200 et 500 µg/mL) **(****Figure 4****).**

### Exemple 4 : Recherche d'un effet immunostimulant de l'extrait d'algues chez la souris naïve

Les effets cliniques de l'extrait d'algues sur la dissémination bactérienne **(****Figure 3D****),** son absence d'effet anti-SAMS *in vitro* **(****Figure 4****)** ainsi que son absence d'action clinique en absence de traumatisme **(****Figures 3E** et **F****)** ont conduit à rechercher un effet immunostimulant du polysaccharide. Une relation dose-effet de l'extrait sur la sécrétion de cytokines pro-inflammatoires (IL-12 par les CDs, TNFα par les macrophages et INF γ par les NK et LT) pulmonaires et spléniques a premièrement été recherchée chez la souris naïve. L'évaluation de la production cytokinique a été réalisée en cytométrie en flux après marquage intracellulaire. Pour cela l'extrait a été injecté à plusieurs temps (2h et 12 heures avant euthanasie) en intrapéritonéal. L'extrait a également été administré en intra-trachéal afin d'essayer d'induire une stimulation directe des cellules de l'immunité muqueuse pulmonaire.

### L'administration intra-péritonéale de l'extrait d'algues 2 heures et 12 heures avant l'euthanasie n'induit pas d'augmentation de la production de cytokines pro-inflammatoires (IL-12, TNFα, INF y) chez la souris naïve

L'extrait d'algues n'induit pas d'augmentation du pourcentage CDs pulmonaires produisant de l'IL-12 **(Figure 5A),** quelle que soit la dose utilisée. Il n'augmente pas la proportion de macrophages produisant du TNFα **(Figure 5B)** ni d'augmentation du nombre de NK **(Figure 5C)** ou de LT INFγ ⁺ dans le tissu pulmonaire. Les nombres totaux de CDs, NK et LT pulmonaires n'étaient pas modifiés par l'injection de l'extrait d'algues chez les souris naïves.

Les effets d'une administration de l'extrait d'algues ont également été évalués 12h après l'injection intra-péritonéale du composé. Les mêmes posologies ont été étudiées. À 12h, on ne notait aucune différence pour la production de cytokines pro-inflammatoires pulmonaires ainsi que pour le nombre de cellules (données non montrées).

### L'administration intra-trachéale de l'extrait d'algues 2 heures et 12 heures avant l'euthanasie n'induit pas d'augmentation de la production de cytokines pro-inflammatoires (IL-12, TNFα, INF y) chez la souris naïve

La voie intra-péritonéale ayant peu d'effets chez les souris naïves sur les cellules immunitaires étudiées, l'effet d'un traitement administré directement par voie intra-trachéale a été testé. La production de cytokines a été évaluée 2h **(T (H-2))** et 12h **(T (overnight))** après l'instillation intra-trachéale de l'extrait d'algues. Aucune différence de sécrétion n'a été mise en évidence comparée aux souris témoins **(Naïves)** et à l'administration de LPS, à la dose de 50µg, 2 heures avant l'euthanasie **(LPS (H-2)) (****Figure 6****).**

### Exemple 5 : Recherche de l'effet immunostimulant de l'extrait d'algues dans un modèle d'immunodépression post-traumatique

L'absence d'effet immunostimulant de l'extrait d'algues chez la souris naïve a conduit à poursuivre les recherches dans un modèle de souris traumatisées et secondairement infectées, reproduisant le scénario observé en clinique. L'effet immunostimulant de l'extrait d'algues a ainsi été évalué dans un modèle d'immunodépression post-traumatique. Le traumatisme crânien était réalisé chez des souris mâles C57/BI6, puis l'extrait d'algues était administré par voie intra-péritonéale à la dose de 200 µg dès deux heures après le traumatisme puis toutes les 12 heures jusqu'à l'euthanasie (3 injections au total). La pneumonie était induite 24 heures après le traumatisme crânien puis les souris étaient euthanasiées 12 heures après la pneumonie. Les souris étaient réparties en 4 groupes : Sham (S), pneumonie seule **(PN),** traumatisme crânien + pneumonie non traitées **(TC+PN)** et traumatisme crânien + pneumonie traitées par l'extrait d'algues **(TC+PN+TX) (****Figure 7****).**

### L'administration de l'extrait d'algues n'augmente pas le pourcentage de cellules de l'immunité innée pulmonaires produisant des cytokines pro-inflammatoires

Dans un modèle de pneumonie post-traumatique, l'administration intra-péritonéale de l'extrait d'algues n'augmente pas la proportion de CDs productrices d'IL-12 **(****Figure 8A****),** de macrophages producteurs de TNFα **(****Figure 8B****)** ni de NK ou LT producteurs d'INFy **(****Figure 8C****).**

### L'administration de l'extrait d'algues augmente le nombre de cellules NK productrices d'interféron γ

Le traumatisme crânien provoque, au niveau du poumon, une diminution du nombre total de cellules NK **(****Figure 9A****),** ainsi qu'une diminution des cellules NK produisant de l'interféron γ (p<0,01) **(****Figure 9B****).** L'administration de l'extrait d'algues induit une augmentation du nombre de cellules NK total et productrices d'interféron γ dans le poumon **(****Figures 9A et 9B****)** (p<0,05) sans augmenter le pourcentage de cellules INFγ⁺ **(****Figure 8C****).** Cet effet n'est pas retrouvé aussi clairement pour les lymphocytes T même si une tendance se dessine (p=0.17) **(****Figure 9C****).**

### Exemple 6 : Recherche in vitro d'un effet direct de l'extrait d'algues sur les cellules NK de souris naïves et traumatisées

### L'extrait d'algues n'a pas d'effet in vitro sur sécrétion d'interféron γ ou l'expression du facteur d'activation KLRG 1 par les cellules NK pulmonaires

Devant l'augmentation du nombre de cellules NK productrices d'interféron γ chez les souris traitées par l'extrait, et l'absence d'augmentation du taux des chimiokines CXCL-1 et CXCL-2, il a été recherché si l'effet retrouvé *in vivo* était dû à une action directe du traitement sur les cellules NK. Pour cela les cellules NK pulmonaires de souris naïves et traumatisées ont été triées magnétiquement puis stimulées *in vitro* durant 5 heures selon 3 conditions : Contrôle (ctrl), extrait d'algues, et PMA + lonomycine (PMA + lono).

À la différence de la stimulation par PMA-lono, la stimulation *in vitro* par l'extrait d'algues n'induit pas d'augmentation la production d'interféron γ **(****Figure 10B****)** ni du marqueur d'activation KLRG 1 par les cellules NK **(****Figure 10C****).** Le nombre de cellules NK n'était pas différent dans les différents groupes après 5 heures de stimulation. **(****Figure 10D****).**

### Exemple 7 : Recherche de l'effet bactériologique en cas de déplétion des cellules NK in vivo

Les souris étaient réparties en 3 groupes : Les souris traumatisées et infectées **(TC + PN),** les souris traumatisées, infectées et déplétées en cellules NK **(TC + PN+ del),** et les souris traumatisées, infectées, déplétées en cellules NK et traitées par l'extrait d'algues **(TC + PN + del + TX).** La pneumonie était induite 24 heures après le traumatisme crânien. L'euthanasie survenait 48h après l'induction de la pneumonie. La déplétion des cellules NK était réalisées 2h et 48h après le traumatisme crânien. Les charges bactériennes spléniques ainsi que et l'efficacité de la déplétion de NK pulmonaire (FACS) étaient analysées à 48h de l'induction de la pneumonie **(****Figure 11****).**

### L'extrait d'algues ne limite pas la dissémination bactérienne splénique chez la souris traumatisée déplétée en cellules NK

Afin d'évaluer si les effets de l'extrait d'algues sur la dissémination bactérienne splénique étaient dus à l'augmentation du nombre de cellules NK pulmonaires, il a été évalué si cet effet sur la bactériémie était retrouvé en cas de déplétion des cellules NK *in vivo.*

En absence de cellules NK pulmonaires (déplétion supérieure à 95%), les effets de l'extrait d'algues sur la dissémination bactérienne splénique à 48h de l'infection ne sont pas retrouvés (TBI + PN+ del vs TBI + PN + del + TX) (Figure 12).

### Exemple 8 : Étude de l'effet de l'extrait d'algues sur la sécrétion de chimiokines spléniques et pulmonaires

L'administration du polysaccharide sulfaté induit une augmentation du nombre de cellules NK pulmonaires chez les souris traumatisées et infectées. Pour savoir si cette augmentation de nombre était due à un recrutement pulmonaire des cellules NK par l'induction d'une sécrétion de chimiokines, les sécrétions spléniques et pulmonaires des chimiokines CXCL1(KC), CCL2(MCP-1), CCL19(MIP-3b), CXCL2(MIP-2), CXCL10(IP-10), CCL8(MCP-2), CCL3(MIP-1a), CCL20(MIP-3a), CCL4(MIP-1b), et CCL21(6Ckine) ont été évaluées en Luminex. Ces chimiokines sont impliquées dans la chimioattraction des cellules NK après une inflammation aigüe.

Les souris étaient réparties en 7 groupes : Les souris naïves (SHAM), les souris traumatisées seules (TC), les souris traumatisées et traitées par le composé (TC + TX), les souris infectées seules (PN) (uniquement pour l'études des chimiokines pulmonaires), les souris infectées et traitées par l'extrait d'algues (PN + TX) (uniquement pour l'études des chimiokines pulmonaires), les souris traumatisées et infectées (TC + PN) et les souris traumatisées, infectées et traitées par l'extrait d'algues (TC + PN + TX). La pneumonie était induite 24 heures après le traumatisme crânien. L'euthanasie survenait 12h après l'induction de la pneumonie. Les taux spléniques et pulmonaires des chimiokines suscitées étaient analysées par technique Luminex (**Figure 13**).

L'extrait d'algues entraine une augmentation des taux spléniques des chimiokines CCL2, CCL3, CCL4 et CCL8 (**Figure 14**). L'extrait d'algues n'a aucun effet sur la sécrétion splénique des chimiokines CXCL1, CCL19, CXCL2, CXCL10, CCL20, CCL4, CCL21 (données non montrées). L'injection intra-péritonéale de l'extrait d'algues n'entraine pas non plus d'augmentation des taux de chimiokines analysées au niveau pulmonaire (données non montrées).

### Conclusions :

Un effet clinique de l'extrait d'algues a initialement été recherché dans un modèle d'infection pulmonaire bactérienne post-traumatique. Aucun effet n'a été mis en évidence sur la charge bactérienne pulmonaire mais ce traitement limitait la dissémination bactérienne systémique à 48 heures de l'infection (charge bactérienne splénique) chez les souris traumatisées. Cet effet est dû à une restauration des fonctions immunitaires paralysées par le traumatisme et non à une activité anti-bactérienne directe, l'extrait d'algues n'ayant pas d'activité anti-SAMS *in vitro* et aucun effet sur la dissémination bactérienne chez la souris infectée non traumatisée.

La diminution de la dissémination bactérienne observée dans le modèle murin de pneumonie post-traumatique après traitement par l'extrait d'algues ainsi que les données préliminaires réalisées *in vitro* ont conduit à rechercher *in vivo* chez la souris naïve une relation dose-effet de l'extrait sur les cellules de l'immunité innée (CDs, macrophages et cellules lymphoïdes NK et LT). Les doses de 50, 200 et 500 µg/ souris ont été choisies en rapport avec des données préliminaires de toxicité réalisée *in vivo* (tests réalisés par le laboratoire Effimune, Nantes, France). Les moments d'injection (2 heures et 12h avant le sacrifice) ont été choisis afin de mettre en évidence une augmentation de la sécrétion d'IL-12, de TNF α et d'INFy mais aucun effet n'a été retrouvé ni dans le poumon ni dans la rate. L'extrait d'algues n'induisait pas non plus d'augmentation d'expression membranaire du CMH II par les cellules dendritiques (données non montrées). En l'absence d'immunodépression, l'administration de l'extrait d'algues n'induit pas de stimulation des cellules de l'immunité innée. Cela corrobore les résultats bactériologiques dans lesquels l'administration de l'extrait d'algues, en absence de traumatisme (et donc d'immunodépression), ne limite pas la dissémination bactérienne, contrairement à ce qu'on observe chez les souris traumatisées et infectées.

Une stimulation de la sécrétion de cytokines pro-inflammatoires a donc été recherchée dans un modèle de souris traumatisées chez qui une pneumonie à SAMS à 24 heures du traumatisme a été induite. L'injection intrapéritonéale de l'extrait d'algues toutes les 12 h (du TC jusqu'à l'euthanasie, soit 3 au total) n'augmentait pas la proportion de cellules de l'immunité innée productrices de cytokines pro-inflammatoires. Cependant, le nombre de cellules NK pulmonaires totales et le sous-groupe produisant de l'INFy (cellules d'intérêt dans le cadre d'une infection pulmonaire aigüe) étaient augmentés dans le groupe de souris traitées par l'extrait marin. Cette augmentation n'était retrouvée que sur le lieu de l'infection (aucune différence retrouvée dans la rate). Le traumatisme crânien induit donc une diminution importante du nombre de cellules NK produisant de l'INFγ, diminution partiellement compensée par l'administration de l'extrait d'algues. Cet effet n'était pas significatif pour les lymphocytes T.

La question s'est donc posée de savoir si une augmentation du nombre de cellules NK pulmonaires est suffisante à elle seule pour expliquer la limitation de dissémination bactérienne à 48 heures de l'infection chez les souris traitées. Les cellules NK sont essentielles à la réponse antibactérienne, notamment en cas d'infection pulmonaire aigüe. De plus, dans cette situation clinique, l'action des cellules NK ne se limite pas à la sécrétion de substances pro-inflammatoires mais entraine une co-stimulation des autres cellules de l'immunité innée, notamment en diminuant l'apoptose de polynucléaires neutrophiles et en préservant leurs capacités fonctionnelles. Les cellules NK présentent également un rôle d'immunomodulation en détruisant les CDs non activées et les macrophages suractivés pouvant causer des dommages tissulaires. Dans ce contexte, l'effet de l'extrait a été évalué sur la dissémination bactérienne splénique dans un modèle d'immunodépression post-traumatique chez la souris déplétée en cellules NK. Les effets de l'extrait sur la charge bactérienne splénique n'étaient pas retrouvés en cas de déplétion de cellules NK (plus de 95% de déplétion de cellules NK pulmonaires), confirmant le rôle de ces cellules dans l'action de l'extrait marin in *vivo.*

La sécrétion de chimiokines en réponse à l'injection de l'extrait d'algues, notamment par les cellules épithéliales via la voie TLR4-NF-kB est une des hypothèses pouvant expliquer l'augmentation du nombre de cellules NK sur les lieux de l'infection. Ainsi, les taux pulmonaires et spléniques des principales chimiokines impliquées dans le chimiotactisme des cellules NK en cas d'inflammation aigüe ont été mesurées : CXCL1(KC), CCL2(MCP-1), CCL19(MIP-3b), CXCL2(MIP-2), CXCL10(IP-10), CCL8(MCP-2), CCL3(MIP-1a), CCL20(MIP-3a), CCL4(MIP-1b), CCL21(6Ckine) avant et après injection de l'extrait d'algues. L'étude de ces chimiokines par technique Luminex a permis de mettre en évidence une augmentation de sécrétion des chimiokines CCL2, CCL3, CCL4 et CCL8 au niveau splénique. Aucun effet de l'injection de l'extrait d'algues n'a été retrouvé ni sur la sécrétion des chimiokines pulmonaires au temps étudié, ni pour les autres chimiokines spléniques.

Plusieurs hypothèses sont ainsi envisageables : 1) le pic de sécrétions de chimiokines pulmonaires n'aurait pas été mis en évidence car plus précoce 2) d'autres voies de signalisation intermédiaires sont impliquées dans l'augmentation du nombre de cellules NK dans les poumons. Les chimiokines CCL2, CCL3, CCL4 et CCL8 sont principalement sécrétées par les cellules épithéliales et endothéliales et permettent le recrutement des cellules immunitaires (neutrophiles, cellules NK, Lymphocytes) après une agression pulmonaire aigüe notamment.

Pour finir, il a été recherché si l'extrait d'algues avait un effet direct sur les cellules NK dont l'expression membranaire ou intracellulaire du récepteur TLR 4 est débattu dans la littérature. Pour cela des cellules NK pulmonaires de souris naïves et traumatisées ont été isolées par tri magnétique et stimulées *in vitro* par l'extrait d'algues en présence d'IL-2. Aucun effet sur la sécrétion d'INFy, sur l'expression membranaire de KLRG1 ainsi que sur le nombre de cellules NK par puits n'a été détecté. Les mécanismes d'action de l'extrait d'algues restent donc à élucider.

## Revendications

1. Extrait d'algues de l'ordre des ulvales comprenant des polysaccharides polyanioniques sulfatés et non sulfatés dont le poids moléculaire est inférieur ou égal à 50 kDa, pour son utilisation pour la prévention et/ou le traitement d'une complication induite par une immunodépression post-traumatique.

2. Extrait d'algues pour l'utilisation selon la revendication 1, pour la prévention et/ou traitement d'une complication septique de l'immunodépression post-traumatique.

3. Extrait d'algues pour l'utilisation selon la revendication 2, où ladite complication septique est une infection nosocomiale, notamment une infection nosocomiale sélectionnées dans le groupe constitué des pneumopathies, telles que les pneumonies acquises sous ventilation mécanique (PAVMs), des infections urinaires, des infections sur cathéters veineux centraux, des infections bactériennes cérébroméningées telles méningites empyèmes et abcès cérébraux.

4. Extrait d'algues pour l'utilisation selon l'une quelconque des revendications 1 à 3, où ladite immunodépression post-traumatique est consécutive à un ou plusieurs traumatismes sévères, en particulier un traumatisme crânien sévère.

5. Extrait d'algues pour l'utilisation selon l'une quelconque des revendications 1 à 4, où ledit extrait d'algues est un extrait d'algues vertes de type *Ulva.*

6. Extrait d'algues pour l'utilisation selon l'une quelconque des revendications 1 à 4, où lesdits polysaccharides polyanioniques sulfatés et non sulfatés de poids moléculaire inférieur ou égal à 50 kDa ont un poids moléculaire inférieur à 15 kDa, et de préférence supérieur à 500 Da.

7. Extrait d'algues pour l'utilisation selon la revendication 6, où l'extrait d'algues ne comprend pas de polysaccharides polyanioniques sulfatés ou non sulfatés dont le poids moléculaire est supérieur à 15 kDa.

8. Extrait d'algues pour l'utilisation selon l'une quelconque des revendications 1 à 7, où l'extrait d'algue comprend :
- du mannose ; et/ou
- de l'arabinose ; et/ou
- du galactose; et/ou
- du glucose; et/ou
- du rhamnose; et/ou
- du xylose; et/ou
- de l'acide glucuronique.

9. Extrait d'algues pour l'utilisation selon l'une quelconque des revendications 1 à 8, où l'extrait d'algue comprend :
- de 10 à 50 % de carbone ;
- de 1 à 10% d'hydrogène ;
- de 1 à 5 % d'azote ;
- de 20 à 50 % d'oxygène ; et
- de 1 à 15 % de soufre ;
en pourcentage massique de la matière sèche totale de l'extrait d'algues.

10. Extrait d'algues pour l'utilisation selon l'une quelconque des revendications 1 à 9, où l'extrait d'algues est obtenu par un procédé de préparation dans lequel:
a) les algues sont lavées et dessablées ;
b) lesdites algues sont broyées ;
c) la phase solide du broyat est séparée de sa phase liquide ;
d) ladite phase liquide est clarifiée ;
e) le jus obtenu à l'étape d) est ultrafiltré sur une membrane de 50 kDa ou moins; et
f) le jus de filtration obtenu à l'étape e) est concentré puis séché.
